# EUROPEAN PATENT SPECIFICATION

(11) **EP 1 554 381 B1**
(45) Date of publication and mention of the grant of the patent: **25.11.2009**
(21) Application number: 03703543.3
(22) Date of filing: 06.02.2003
(51) Int. Cl.: C12N 15/11, A61K 31/7088

(54) **POST TRANSCRIPTIONAL SILENCING OF TISSUE FACTOR EXPRESSION BY SHORT INTERFERING RNAS**
POST-TRANSCRIPTIONAL SILENCING DER GEWEBEFAKTOR EXPRESSION DURCH KURZE INTERFERIERENDE RNAS
SUPPRESSION POST TRANSCRIPTIONNELLE DE L'EXPRESSION DU FACTEUR DE TISSU PAR DES ARN A INTERFERENCES COURTES

(30) Priority: 07.02.2002 NO 20020612; 08.02.2002 US 354515 P; 16.10.2002 NO 20024987
(43) Date of publication of application: 20.07.2005
(73) Proprietor: siRNAsense AS, 0349 Oslo (NO)
(72) Inventor: PRYDZ, Hans Peter B., 0376 Oslo (NO); HOLEN, Torgeir, 0486 Oslo (NO); AMARZGUIOUI, Mohammed, 0364 Oslo (NO); WIIGER, Merete, 3408 Tranby (NO); BABAIE, Eshrat, 1338 Sandvika (NO)
(74) Representative: Baldock, Sharon Claire
(86) International application number: PCT/NO2003/000045
(87) International publication number: WO 2003/066650

(56) References cited:
- WO-A-00/38517
- WO-A-03/020111
- JANKOWSKY ECKHARD ET AL: "Oligonucleotide facilitators enable a hammerhead ribozyme to cleave long RNA substrates with multiple-turnover activity" EUROPEAN JOURNAL OF BIOCHEMISTRY, vol. 254, no. 1, May 1998 (1998-05), pages 129-134, XP002270885 ISSN: 0014-2956 cited in the application
- HOLEN TORGEIR ET AL: "Positional effects of short interfering RNAs targeting the human coagulation trigger Tissue Factor" NUCLEIC ACIDS RESEARCH, vol. 30, no. 8, 15 April 2002 (2002-04-15), pages 1757-1766, XP002270886 ISSN: 0305-1048
- AMARZGUIOUI MOHAMMED ET AL: "Tolerance for mutations and chemical modifications in a siRNA." NUCLEIC ACIDS RESEARCH, vol. 31, no. 2, 15 January 2003 (2003-01-15), pages 589-595, XP002270887 ISSN: 0305-1048 (ISSN print)
- CAPLEN ET AL.: "Specific inhibition of gene expression by small double-stranded RNAs in invertebrate and vertebrate systems" PNAS, vol. 98, no. 17, 14 August 2001 (2001-08-14), pages 9742-9747, XP002232936

## Description

The present invention relates to synthesised RNAs, more specifically short interfering RNAs (siRNAs) that are able to modulate the expression of Tissue Factor (TF), as well as to a pharmaceutical preparation comprising the synthesised siRNA(s) and use thereof.

### Background of the invention

Mechanisms that silence unwanted gene expression are critical for normal cellular function, and RNA silencing is a new field of research that has coalesced during the last decade from independent studies on various organisms. It has been known for a long time that interactions between homologous DNA and/or RNA sequences can silence genes and induce DNA methylation (1). The discovery of RNA interference (RNAi) in *C*. *elegans* in 1998 focused attention on double-stranded RNA (dsRNA) as an elicitor of gene silencing, and many gene-silencing effects in plants are now known to be mediated by dsRNA (1).

RNAi is usually described as a posttranscriptional gene-silencing (PTGS) phenomenon in which dsRNA trigger degradation of homologous mRNA in the cytoplasm (1). However, the potential of nuclear dsRNA to enter a pathway leading to epigenetic modifications of homologous DNA sequences and silencing at the transcriptional level should not be discounted. Also, even though the nuclear aspects of RNA silencing have been studied primarily in plants, there are indications that similar RNA-directed DNA or chromatin modifications might occur in other organisms as well.

RNAi in animals, and the related phenomena of PTGS in plants, result from the same highly conserved mechanism, indicating an ancient origin (1). The basic process involves a dsRNA that is processed into shorter units (called short interfering RNA; siRNA) that guide recognition and targeted cleavage of homologous messenger RNA (mRNA). The dsRNAs that (after processing) trigger RNAi/PTGS can be made in the nucleus or cytoplasm in a number of ways.

The processing of dsRNA into siRNAs, which in turn degrade mRNA, is a two-step RNA degradation process. The first step involves a dsRNA endonuclease (ribonuclease III-like; RNase III-like) activity that processes dsRNA into sense and antisense RNAs which are 21 to 25 nucleotides (nt) long, i.e. siRNA. In Drosophila this RNase III-type protein is termed Dicer. In the second step the antisense siRNAs produced combine with, and serve as guides for, a different ribonuclease complex called RNA-induced silencing complex (RISC), which cleaves the homologous single-stranded mRNAs. RISC cuts the mRNA approximately in the middle of the region paired with the antisense siRNA, after which the mRNA is further degraded. dsRNAs from different sources can enter the processing pathway leading to RNAi/PTGS. Furthermore, recent work also suggests that there may be more than one pathway for dsRNA cleavage, producing distinct classes of siRNAs that may not be functionally equivalent.

RNA silencing (which is active at different levels of gene expression in the cytoplasm and the nucleus) appears to have evolved to counter the proliferation of foreign sequences such as transposable elements and viruses (many of which produce dsRNA during replication). However, as RNAi/PTGS produce a mobile signal that induces silencing at distant sites, the possibility of injecting directly siRNAs to shut down protein synthesis and/or function as a therapeutic tool in mammalian cells should be considered.

So far, little is known about general effects of mutations or chemical modifications in a siRNA sequence. Boutla et al. (14) reported that a mutated siRNA with a single centrally located mismatch relative to the mRNA target sequence retained substantial activity in *Drosophila.* In contrast, Elbashir et al. (15) found that a single mismatch was deleterious to activity in an *in vitro Drosophila* embryo lysate assay. In the present application we have tried to reconcile these two conflicting results by depicting the RNAi process in vivo as a dynamic process where several factors influence the final outcome, among them siRNA target position, siRNA concentration, mRNA concentration, mRNA production and siRNA's inherent cleavage activity, an activity that can be gradually reduced by mismatch mutations.

Some other results have also been reported. For example, Jacque et al (16) find that a single mismatch in a siRNA targeting HIV's LTR did lose only some activity, while another siRNA targeting HIV's VIF lost almost no activity at all. Four mutations, however, abolished activity completely. Other instances of complete abolishment of activity is seen by Gitlin et al (17), Klahre et al (18) and Garrus et al (19), using 5, 6 and 7 mutations respectively. A central, double mutations used by Boutla and our own group (14, 15), led to severe activity loss also for Yu et al (20) and Wilda et al (21), the latter using a siRNA with only 17 basepairs. Interestingly, in view of our very active end-methylated siRNAs, is Tuschl's report that fully 2'-OH methylated siRNA are inactive.

Further, two published reports of abolishment of activity by a single mutation exist. One of them, however, the work by Brummelkamp et al (22), is using a short hairpin RNA (shRNA) that is assumed to produce siRNA by action of Dicer (23). This shRNA construct was inactivated either by a single mutation in the putative second nucleotide of the shRNA, or by a single mismatch in the putative ninth nucleotide. Gitlin et al (17), on the other hand, argued the case for single mutation inactivation more strongly by isolating siRNA resistant polio virus strains containing a single mutation in the target site on the genomic RNA, either in the sixth nucleotide of the siRNA or the ninth nucleotide, both counted from the 5' end of the sense strand. On balance, different siRNA seem to be inactivated to different degrees.

Traditionally, chemical modification of nucleic acids has *inter alia* been used to protect single stranded nucleic acid sequences against nuclease degradation and thus obtaining sequences with longer half life. For example, WO 91/15499 discloses 2'O-alkyl oligonucleotides useful as antisense probes. Also, 2-O-methylation has been used to stabilize hammerhead ribozymes (4). However, little is known about the effects of chemical modifications of siRNAs. Further, the presence of large substituents in the 2'hydroxyl of the 5'terminal nucleotide might interfere with the proper phosphorylation of the siRNA shown to be necessary for the activity of the siRNA (24).

Thus, an inherent differential activity in the various siRNAs in a population would mean that different siRNAs will be affected by siRNA modifications, chemical or mutational, in different ways.

Tissue Factor (TF) is the most potent trigger of blood coagulation (2) and instrumental in causing arterial thrombosis upon rupture of atherosclerotic plaques. There is also good evidence that high content of TF in cancer cells correlates with cancer-driven angiogenesis and with tendency to metastasis. Furthermore, TF is of central pathogenic importance in case of septic disseminated intravascular coagulation (e.g. meningococcal sepsis). Thus, methods to modulate or silence TF would be of great value.

Patent application WO 01/75164 (A2) discloses a Drosophila in vitro system which is used to demonstrate that dsRNA is processed to RNA segments 21-23 nucleotides (nt) in length, wherein these 21-23 nt fragments are specific mediators of RNA degradation. Caplen et al. (28) reports that synthetic siRNA directed towards the CAT gene and *C. elegans unc*-22 gene reduced the expression in vertebrate and invertebrate systems respectively. However, neither WO 01/75164 nor Caplen et al. (28) disclose anything regarding siRNAs which are able to directly modulate the expression of TF in mammals. Janowsky and Schwenzer (26) reports that the activation of a hammerhead ribozyme by oligonucleotide facilitators exampled *inter alia* with a hammerhead ribozyme construct and oligonucleotide facilitators directed towards hTF. However, the mechanism to inhibit gene expression with hammerhead ribozymes and oligonucleotide facilitators as utilized by Janowsky and Schwenzer (26) are clearly different from the mechanism by which siRNAs inhibit the expression of any gene such as the TF coding gene.

Apart from preliminary studies on antibodies, no clinically useful direct inhibitor of TF is available, nor can it be usefully regulated at the level of gene expression. Studies on silencing of transgenes in plants has led to a rather general opportunity for suppressing gene expression, and dsRNA is already established as a routine tool for gene silencing in e.g. plants, *C*. *elegans* and Drosophila (3). However, dsRNA cannot be used in mammalian cells because of unspecified effects. Furthermore, even though all gene expression can, in principle, be suppressed by use of e.g. oligonucleotide (synthetic chains), ribozymes or siRNA molecules, it is extremely hard to find exactly what part of an mRNA sequence that should be used in order to synthesise siRNA(s) which are active in suppressing a specific gene as siRNAs are here heavily position-dependent. This aspect is further supported by the results reported by Harborth et al. (31), which experienced that without revealing any unusual features, siRNA-sequences directed towards different sequences of the same gene exerted quite dissimilar efficiency. In addition, as sites on the mRNA target can also be differentially accessible to ribozymes (4), efforts to identify really efficient ribozymes towards TF with little or no toxicity, has not yet succeeded. Thus, it is a hope that current efforts to exploit RNAi in mammals may lead to novel developments in gene therapy (5), and that siRNAs may provide a tool in the specific silencing of mammalian TF.

### Summary of the invention

It is therefore an object of the present invention to provide siRNA that, together with RISC, are able to directly modulate the expression of TF in mammals. These objects have been obtained by the present invention, characterised by the enclosed claims. Generally, the present invention relates to short interfering RNA molecules which are double or single stranded and comprise at least 21-25 nucleotides, and wherein said siRNAs are able to modulate the gene expression of TF.

siRNAs are dsRNAs of ≈ 21-25 nucleotides that have been shown to function as key intermediates in triggering sequence-specific RNA degradation. In the work with siRNAs and the coagulation trigger TF, the present inventors show that siRNAs can bypass the RNAse III-like RNAi initiator Dicer and directly charge the effector nuclease RISC so that TF mRNA is degraded. According to the present invention it is shown that different siRNAs against the same target vary in efficiency, and thus, siRNAs are synthesised against different parts of TF mRNA, after which they combine with RISC which is then guided for specific degradation/silencing of TF mRNA.

Also, the siRNA of the present invention may comprise one or two base-pairing mutations compared to the wild type sequence of the present siRNA molecules, i.e. the modified sequence are about 90% homologous to the wild type siRNA molecules of the present invention.

The present invention also disclose siRNA molecules which are chemically modified compared to the wild type siRNA of the present invention, i.e. by the introduction of a lower alkyl, such as methyl in the 2'OH-position or by the introduction of phosphorothioate linkages in the sequence.

The present invention further discloses that siRNA silencing is relatively stable but declines over time and that TF coagulation activity can be reduced five-to-ten-fold and remain so over a period of 5 days (120 hours) after a single siRNA transfection. Thus, the present invention also relates to a pharmaceutical preparation comprising the siRNAs of the present invention, as well as use of the pharmaceutical preparation. According to the knowledge of the inventors, the present invention discloses for the first time siRNA silencing of gene expression underlying a complex physiological system in mammalian cells. The positional effects observed will make the search for efficient siRNAs more demanding, but when identified, efficient siRNAs show great promise for in future *in vivo* studies.

### Detailed description of the invention.

More specific, the present inventions disclose short interfering RNA molecules (siRNAs), **characterized in that** it is a double or single stranded siRNA comprising at least 19 nucleotides, and which is directed towards a tissue factor (TF) coding nucleic acid sequence or fragments thereof, and wherein the siRNA molecule is selected from the group consisting of
(a) a siRNA molecule having the nucleic acid sequence depicted in SEQ ID NO 1, 2, 3, 4, 6, 7, 8, 10 or 11
(e) a siRNA having the nucleic acid sequence in (a) - (d) wherein the sequences are modified by the introduction of a C₁-C₃-alkyl, C₁-C₃-alkenyl or C₁-C₃-alkylyl group in one or more of the 2' OH hydroxyl group in the sequence and/or by replacing the phosphodiester bond with a phosphorothioate bond.

It is preferred according to one aspect that the siRNAs of the present invention are double stranded. According to another aspect, the siRNAs of the present invention are 21-25 nucleotides long, more preferably 21 nucleotides long and even more preferably identified by SEQ ID NOS: 1-4, 6-8 and 10-11.

According to still another aspect of the invention, the siRNAs are directed to TF or fragments thereof which are of vertebrate origin, preferably mammalian origin, more preferably human origin.

Further it is preferred that the present siRNAs induces cleavage of mRNA which event more preferably identified by SEQ ID NO 1 or SEQ ID NO 2.

Also described herein are siRNA molecules which comprise a sequence which is about 90 % homologue to a siRNA molecule depicted in SEQ ID NO 1 to SEQ ID NO 8 or that the siRNA comprises a sequence as depicted in SEQ ID NO 1 to SEQ ID NO 8 wherein a C₁-C₃-alkyl, C₁-C₃-alkenyl or C₁-C₃-alkylyl group is introduced in one or more of the 2' OH hydroxyl group. Preferably, the siRNA molecule has the sequence as depicted in SEQ ID NO 9 to SEQ ID NO 11).

Further, according to another aspect it is preferred that the siRNA molecules of the present invention comprise a sequence as depicted SEQ ID NO 22 to SEQ ID NO 25 wherein the phosphodiester bond has been replaced by a thiophosphodiester bond. Preferably, the modified sequence is the sequence SEQ ID NO 24, 28 or 29, more preferably SEQ ID NO 29.

A method to treat, prevent or inhibit unwanted blood coagulation wherein a pharmaceutical preparation comprising one or more of the siRNAs according to the present invention is administered to an animal or a person in need of such treatment is also described. Preferably, the preparation comprises one or more of the SEQ ID NO 1-4 or 6-8, more preferably it comprises SEQ ID NO 1 and/or SEQ ID NO 2.

In still another aspect, the present invention relates to pharmaceutical preparations comprising one or more of the siRNAs according to the present invention. Preferably, the preparation comprises the siRNAs depicted in SEQ ID NO 1 or 2. It is also preferred that the preparation according to the invention comprises one or more of the sequences depicted in SEQ IN NO 10, 11, 24, 28 or 29, more preferably SEQ ID NO 29. The preparation according to present invention furthermore comprises e.g. diluents, lubricants, binders, carriers, disintegration means, absorption means, colourings, sweeteners and/or flavourings. It is also favourable that the present preparation comprises adjuvants and/or other therapeutical principles.

Further, in still another aspect, the present preparation may be administered e.g. parenterally (e.g. by subcutaneous, intravenous, intramuscular or intraperitoneal injection or infusion), orally, nasally, buccally, rectally, vaginally and/or by inhalation or insufflation. More preferably, the present preparation is formulated as e.g. infusion solutions or suspensions, an aerosol, capsules, tablets, pills, spray, suppositories etc., in dosage formulations containing conventional non-toxic pharmaceutically-acceptable carriers, adjuvants and/or vehicles. The preparation of the present invention may preferably be administered in one dose, in divided doses or by way of sustained release devices, preferably alone or together with other pharmaceuticals.

Further, the present invention also relates the use of one or more of the present siRNAs to prepare a pharmaceutical preparation suitable for the treatment, prevention and/or inhibition of unwanted blood coagulation in vertebrates, preferably mammals, more preferably humans. Preferably, the preparation prepared according to the use of the present invention may be administered e.g. parenterally (e.g. by subcutaneous, intravenous, intramuscular or intraperitonal injection or infusion), orally, nasally, buccally, rectally, vaginally and/or by inhalation or insufflation.

Finally, the present invention also disclose a method to use the pharmaceutical preparation according to the present invention wherein the said pharmaceutical preparation suitable for the treatment, prevention and/or inhibition of unwanted blood coagulation is administered to an animal or person in need of such treatment. The administration route according to the method of the present invention is e.g. parenterally (e.g. by subcutaneous, intravenous, intramuscular or intraperitoneal injection or infusion), orally, nasally, buccally, rectally, vaginally and/or by inhalation or insufflation.

The present invention will now be described in more detail, with reference to figures and examples.

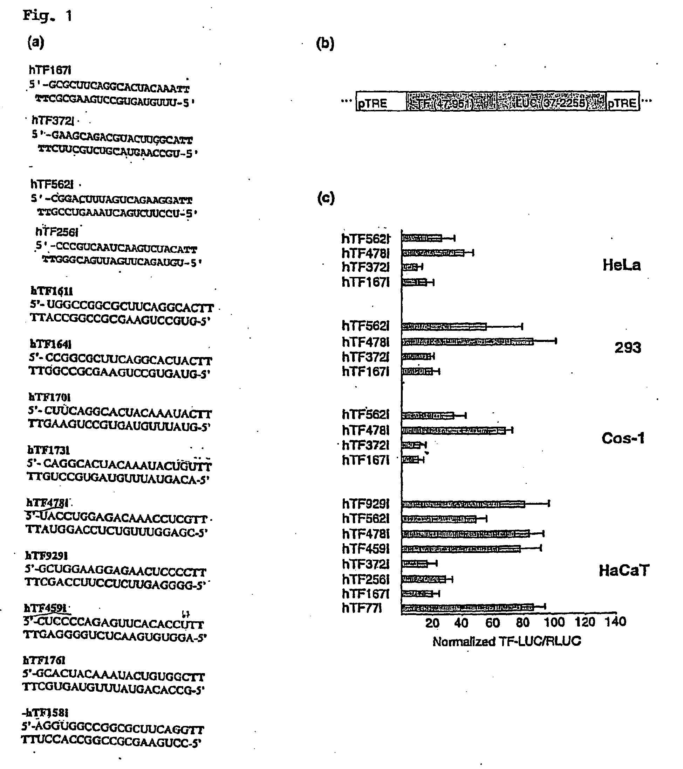

Figure 1 siRNAs, reporter construct and RNAi of transgene expression; a) The sense (top) and antisense (bottom) strands of siRNA species targeting eight sites within human TF (Genbank entry Ace. No. M16553) mRNA are shown, b) Luciferase reporter construct of human TF and c) RNAi by siRNA in cotransfection assays (averages of three or more independent experiments each in triplicate, ± s.d. are shown).

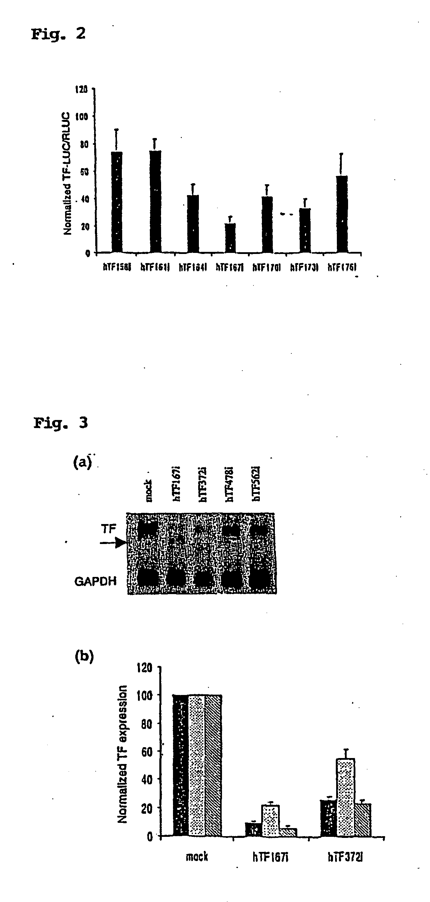

Figure 2 Efficacy of the siRNAs in standard cotransfection assays in HaCaT cells. Different synthetic batches of the hTF167i siRNA showed similar efficacy. Results are averages of at least three experiments, each in triplicate.

Figure 3 siRNA mediated reduction of endogenous TF expression; a) hTF167i and hTF372i induced cleavage of mRNA in transfected cells. The Northern analysis of TF mRNA was performed after transfection of HaCaT cells with siRNA (100 nM) with GADPH as control. Arrowhead indicates cleavage fragments resulting from siRNA action, b) Measurements of the effect of siRNAs on steady state mRNA levels (filled bars), procoagulant activity (dotted bars) and TF protein (antigen) expression (hatched bars) show that siRNA reduces mRNA, TF antigen levels and procoagulant activity. For measurement of procoagulant activity and antigen, cells were harvested 48 h after si transfection to accommodate the 7-8 h half-life of TF protein. Data are from a representative experiment in triplicate.

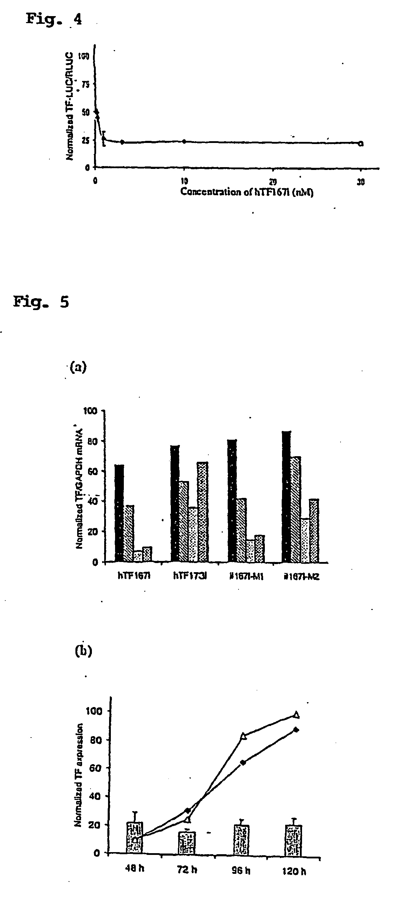

Figure 4 Dose-response curve for hTF167i.

Figure 5 Time-dependence of siRNA-mediated RNAi; a) Inhibitory activity is reduced when mutations (M1 and M2 refer to one and two mutations, respectively) are introduced into the siRNAs. Cells were transfected with 100 nM siRNA and harvested for mRNA isolation 4, 8, 24 and 48 h (filled bars, lined bars, white bars with black dots and hatched bars, respectively). Expression levels were normalised to GADPH and standardised to mock-transfected cells at all time-points, b) Time-course of decay of inhibitory effect for mRNA levels (closed diamonds), reporter gene activity (open triangles) and procoagulant activity (filled bars).

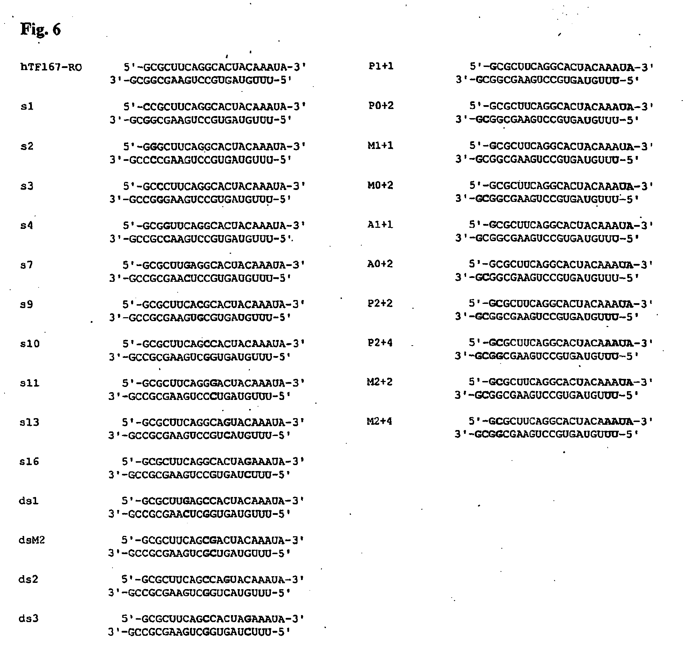

Figure 6 siRNA modifications. (A) Mutated and wild type versions of the siRNA hTF167i. The sequence of the sense strand of wild type (wt) siRNA corresponds to position 167-187 in human Tissue Factor (Ass. No. M16553). Single (s1, s2, s3, s4, s7, s10, s11, s13, s16) and double mutants (ds7/10, ds10/11, ds10/13, ds10/16) are all named according to the position of the mutation, counted from the 5'end of the sense strand. All mutations (in bold) are GC inversions relative to the wild type. (B) Chemically modified versions of the siRNA hTF167i. Non-modified ribonucleotides are in lower case. Phosphorothioate linkages are indicated by asteriscs (*), while 2'-O-methylated and 2'-*O*-allylated ribonucleotides are in normal and underlined bold upper case, respectively.

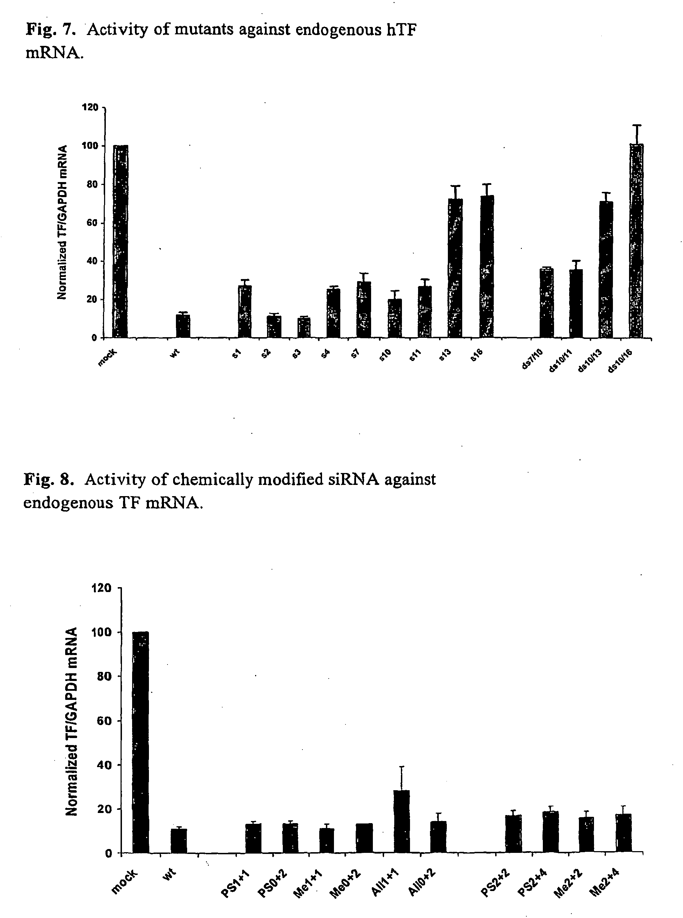

Figure 7 Activity of mutants against endogenous hTF mRNA. HaCaT cells were harvested for mRNA isolation 24h post-transfection. TF expression was normalised to that of GAPDH. Normalised expression in mock-transfected cells was set as 100%. Data are averages + s.d. of at least three independent experiments.

Figure 8 Activity of chemically modified siRNA against endogenous TF mRNA. Experiments were performed and analysed as described in figure 7.

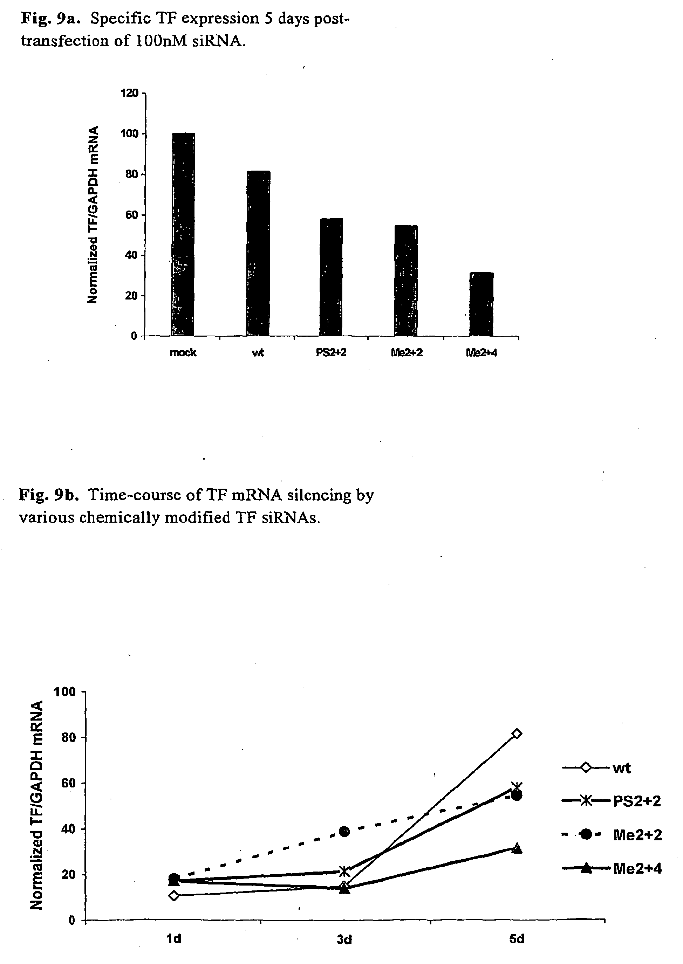

Figure 9 Persistence of TF silencing by chemically modified siRNAs. A) Specific TF expression 5 days post-transfection of 100nM siRNA. B) Time-course of TF mRNA silencing. Cells harvested 1-3-5 days after single transfection of 100nM siRNA. Medium was replaced every second day.

In order to provide the siRNAs to obtain silencing of human TF (hTF), 21-nucleotide RNAs were chemically synthesised using phoshoramidites (Pharmacia and ABI). Deprotected and desilylated synthetic oligoribonucleotides were purified on reverse phase HPLC. Ribonucleotides were annealed at 10 µM in 500µl 10mM Tris-HCl pH 7.5 by boiling and gradual cooling in a water bath. Successful annealing was confirmed by non-denaturing polyacrylamide gel electrophoresis. siRNA species were designed each targeting a site within human TF (Acc.No. M16553) mRNA designed with 2 nucleotides deoxythymidine 3' overhangs and named according to the position of the first nucleotide of the sense strand, using the numbering of the above Genbank entry (Figure 1a). According to the present invention thirteen siRNAs against hTF mRNA (6) were synthesised (Fig.1a). Eight of these (the eight first) siRNAs are termed SEQ ID NO 1 to SEQ ID NO 8, respectively, and some of the sites chosen on the hTF gene were the two most accessible (SEQ ID NO 1 and 2) and the two least accessible (SEQ ID NO 3 and 4) in our previously described ribozyme assay (4). The present invention relates to the synthesised siRNAs according to SEQ ID NO 1-4, 6-8, 10-11 and 22-31.

In order to determine whether mutations were equally tolerated within the whole siRNA, the siRNAs according to the present invention was mapped more systematically. To avoid affecting the duplex stability of the siRNA, only GC pairs were targeted for mutation, by inversion of the pairs as described in example 5 below.

Moreover, the effect of siRNAs in human cells is transient and typically wears off in a few days in cell culture (NO 2002 0612, 29,30). The ability to extend the period of effective silencing would be of great importance for the eventual use of siRNAs as therapeutics. We therefore sought to increase the intracellular stability of siRNA, without compromising activity, by introduction of gradually increasing amounts of various chemical modifications in both ends of the siRNA. We have previously used thiophosphate linkages and 2'-*O*-modifications in the form of methylation and allylation (4) to stabilize hammerhead ribozymes, and decided to explore the feasibility of a similar strategy for stabilization of siRNAs.

The reporter constructs of human TF to be used in the Dual Luciferase system (Promega) were designed using the coding region of TF which were cloned in-frame with the Firefly luciferase (LUC) gene, producing the fusion construct TF-LUC (Acc. No. AF416989). Numbering of the fusion construct refers to that of the genbank entry for TF and to the pGL3-enhancer plasmid (Promega) for LUC. The plasmid pcDNA3-Rluc (Acc. No. AF416990), encoding Renilla luciferase (Rluc; not shown) was used as internal control. Regions of TF and LUC cDNA contained within the construct are indicated in Figure 1b. The Dual Luciferase system is a reporter system which is used to detect how much TF mRNA that is degraded by siRNA(s).

HeLa, Cos-1 and 293 cells were maintained in Dulbecco's Minimal Essential Medium (DMEM) supplemented with 10% fetal calf serum (Gibco BRL). The human keratinocyte cell line HaCaT was cultured in serum free keratinocyte medium supplemented with 2,5 ng/ml epidermal growth factor and 25 µg/ml bovine pituitary extract. All cell lines were regularly passaged at sub-confluence. The day before the experiment cells cultured in DMEM were trypsinized and resuspended in full medium before plating. HaCaT cells were trypsinized until detachment. Trypsin inhibitor was then added and the cells centrifuged for 5 min at 400x g before resuspension in supplemented medium and plating. Cells were transfected one or two days later.

Lipofectamine-mediated transient co-transfections were performed in triplicate in 12-well plates with 0,40 µg/ml plasmid (0,38 µg/ml reporter and 20 ng/ml control) and typically 30 nM siRNA (0,43 µg/ml) essentially as described (4). Luciferase activity levels were measured on 25 µl cell lysate 24 h after transfection using the Dual Luciferase assay (Promega). Serial transfections were performed by transfecting initially with 100 nM siRNA, followed by transfection with reporter and internal control plasmids before harvest time points.

For Northern analyses, HaCaT cells in 6-well plates were transfected with 100 nM siRNA in serum-free medium. Lipofectamine2000™ was used for higher transfection efficiency. Poly(A) mRNA was isolated 24 h after transfection using Dynabeads oligo(dT)₂₅ (Dynal). Isolated mRNA was fractionated for 16-18 h on 1,3% agarose/formaldehyde (0,8 M) gels and blotted on to nylon membranes (Magna-Charge, Micron Separations Inc.). Membranes were hybridised with random-primed TF (position 61-1217 in cDNA) and GAPDH (1,2 kb) cDNA probes in PerfectHyb hybridisation buffer (Sigma) as recommended by the manufacturer.

For TF activity measurements HaCaT cell monolayers were washed thrice with ice-cold barbital buffered saline (BBS) pH 7,4 (BBS, 3 mM sodium barbital, 140 mM NaCl) and scraped into BBS. Immediately after harvesting and homogenisation the activity was measured in a one-stage clotting assay using normal citrated platelet poor plasma mixed from two donors and 10 mM CaCl₂. The activity was related to a standard (6, 7). One unit (U) TF corresponds to 1,5 ng TF as determined in the TF ELISA (6, 7). The activity was normalised to the protein content in the cell homogenates, as measured by the BioRad DC assay.

TF antigen was quantified using the Imubind Tissue Factor ELISA kit (American Diagnostica, Greenwich, CT, USA). This ELISA recognises TF apoprotein, TF and TF:Coagulation Factor VII (FVII) complexes. The samples were left to thaw at 37° C and homogenised. An aliquot of each homogenate (100 µl) was diluted in phosphate-buffered saline containing 1% BSA and 0,1% Triton X-100. This sample was then added to the ELISA-well and the procedure from the manufacturer followed. The antigen levels were normalised to the total protein content in the cell homogenates.

All the various mutant siRNAs were analysed for depletion of endogenous TF mRNA in HaCaT cells, 24h after LIPOFECTAMINE2000-mediated transfection, as described previously for the wild type siRNA sequences of the present invention.

The siRNAs according to the present invention may be used to silence mammalian TF and thereby prevent unwanted blood coagulation. Thus, the present invention also relates to the use of siRNA(s) according the present invention in order to prepare a pharmaceutical preparation and in accordance with techniques well-known in the art for pharmaceutical formulation, which is suitable for the treatment, prevention and/or inhibition of unwanted blood coagulation. The preparation according to the present invention may thus comprise one or more of the siRNAs according to the present invention, diluents, lubricants, binders, carriers disintegration and/or absorption means, colourings, sweeteners flavourings etc., all known in the art. Furthermore, the present preparation may also comprise adjuvants and/or other therapeutical principles, and may be administered alone or together with other pharmaceuticals.

A pharmaceutical preparation according to the present invention may be administered e.g. parenterally (e.g. by subcutaneous, intravenous, intramuscular or intraperitoneal injection or infusion of sterile solutions or suspensions), orally (e.g. in the form of capsules, tablets, pills, suspensions or solutions), nasally (e.g. in form of solutions/spray), buccally, rectally (e.g. in the form of suppositories), vaginally (e.g. in the form of suppositories), by inhalation or insufflation (e.g. in the form of an aerosol or solution/spray), via an implanted reservoir, or by any other suitable route of administration, in dosage formulations containing conventional non-toxic pharmaceutically-acceptable carriers, adjuvants and/or vehicles. The pharmaceutical preparation may further be administered in one dose, in divided doses or by way of sustained release devices.

### EXAMPLES

The invention will now be described by way of examples. Although the examples represent preferred embodiments of the present inventions, they are not to be contemplated as restrictive to the scope of the present invention.

In order to obtain siRNAs that provide silencing of human TF, 21-nucleotide RNAs according to SEQ ID NO 1 to SEQ ID NO 8 were chemically synthesised using phoshoramidites (Pharmacia and ABI). Deprotected and desilylated synthetic oligoribonucleotides were purified on reverse phase HPLC. Ribonucleotides were annealed at 10 µM in 500µl 10mM Tris-HCl pH 7,5 by boiling and gradual cooling in a water bath. Successful annealing was confirmed by non-denaturing polyacrylamide gel electrophoresis. siRNA species were designed each targeting a site within human TF (Acc.No. M16553) mRNA, designed with 2 nucleotides deoxythymidine 3' overhangs and named according to the position of the first nucleotide of the sense strand, using the numbering of the above Genbank entry (Figure 1a).

In conclusion it is demonstrated, in mammalian cells, that double-stranded siRNA synthesised to be complementary to a certain partial sequence on the targeted TF mRNA sequence induces degradation of this specific mRNA (see Example 1). This effect was highly sequence-dependent, and contrary to data in lower organisms, as only a few sites on the TF mRNA were highly susceptible to the corresponding siRNAs. As can be seen from Example 2, the depletion of TF mRNA results in marked reduction of TF protein and procoagulant activity which, according to the knowledge of the present inventors, is the first demonstration of a reproducible such effect on TF not accompanied by toxic side effects. Today it is not possible to base the selection of a susceptible target sequence on the current information available on predicted 3-dimensional structures of RNA protein complexes. However, according to the present invention two positions of satisfactory susceptibility against TF mRNA are provided.

Also, it is demonstrated that a wide range of mutational and chemical modifications of our best siRNA candidate hTF167i are well tolerated. The chemical modifications did show some loss of activity with allyl-modifications to the 5' end, and some, expected, toxicity with longer stretches thio-phosphates, but the siRNA with 2'O-methylated ends do both show strong activity and increased life-span in time-course experiments.

### Example 1 Analysis of hTF siRNA efficacy in cells transiently cotransfected with hTF-LUC and hTF siRNA (i.e. analysis of RNAi by siRNA(s) in cotransfection assays)

The initial analysis of TF siRNA efficacy was performed in HeLa cells transiently cotransfected with hTF-LUC (Fig.1b) and hTF siRNA (Fig.1a) using the Dual Luciferase system (Promega). Ratios of LUC to Rluc expression were normalised to levels in cells transfected with a representative irrelevant siRNA, Protein Serine Kinase 314i (PSK314i).

The siRNAs had potent and specific effects in the cotransfection assays, with the best candidates, hTF167i and hTF372i, resulting in only 10-15 % residual luciferase activity in HeLa cells (Fig.1c). Furthermore, also a positional effect was found, as hTF562i showed only intermediate effect, and hTF478i had very low activity. This pattern was also found in 293, COS-1 and HaCaT cells (Fig.1c), and with siRNAs from different synthetic batches and at various concentrations (the siRNAs caused the same degree of inhibition over a concentration range of 1-100 nM in cotransfection assays; data not shown).

Coculturing siRNA transfected cells with reporter plasmid transfected cells, both in HeLa cells and in the contact-inhibited growth of HaCaT cells, gave no indication of siRNA transfer between cells (data not shown), despite the medium-mediated transfer previously reported by other investigators (8).

### Example 2 Investigation of siRNA position-dependence at codon-level resolution

The accessibility of the region surrounding the target site of the best siRNA (i.e. hTF167i) at a higher resolution was investigated. siRNAs (hTF158i, hTF161i, hTF164i, hTF170i, hTF173i and hTF 176i) were synthesized which targeted sites shifted at both sides of hTF167i in increments of 3 nts, wherein each of them shared 18 out of 21 nts with its neighbours (see Fig. lc). Surprisingly it was found that despite the minimal sequence and position-differences between these siRNAs, they displayed a wide range of activities (Figure 2). There was a gradual change away from the full activity of hTF167i that was more pronounced for the upstream siRNAs. The two siRNAs hTF158i and hTF161i were shifted only nine and six nucleotides away, respectively, from hTF167i, yet their activity was severely diminished. These results suggest that local factor(s) caused the positional effect.

### Example 3 Analysis of hTF siRNA efficacy on endogenous mRNA

The results of cotransfection assays involving the use of forced expression of reporter genes as substrates may be difficult to interpret. The effect of siRNA was therefore also measured on endogenous mRNA targets in HaCaT cells (Fig. 3a) which express TF constitutively. The two best TF siRNAs, hTF167i and hTF372i, showed strong activity also in this assay, as normalised TF mRNA was reduced to 10% and 26%, respectively (Fig. 3a). Interestingly, cleavage products, whose sizes were consistent with primary cleavages at the target sequences, were clearly visible below the depleted main band, though cleavage assays of mRNA based on RNAi have so far failed in mammalian systems (9). Thus, the present invention also relates to siRNA which is able to cleave mRNA in mammalian cells. Furthermore, the observed effect suggests that RISC may be active also in mammals. The third best siRNA in cotransfection assays, hTF256i, also resulted in significant depletion of TF mRNA levels (57% residual expression, data not shown). The remaining TF siRNA did not show any activity as measured by Northern assays (Fig. 3b), nor did they stimulate TF expression, a point of some interest, as transfection with chemically modified ribozymes can induce TF mRNA three-fold (data not shown). Thus, this relative inertness of irrelevant siRNAs (i.e. siRNAs with «non-specific» effects) further enhances the promise of siRNA-based drugs.

The coagulation activity in the HaCaT cells was reduced 5-fold and 2-fold, respectively, in cells transfected with hTF167i and hTF372i, compared to mock-transfected cells (Fig.3b and Fig.5b). The effect of siRNAs on total cellular TF protein was also measured (Fig. 3b), and demonstrated an inhibitory effect that was generally greater than the observed effect on procoagulation activity. Thus, according to the present invention, we conclude that the siRNAs hTF167i and hTF372i display specificity and potency in a complex physiological system, and that we have demonstrated positional effects, as other siRNA molecules against the same target mRNA are basically inactive. Data from a new series of TF siRNA are in support of this conclusion (data not shown), and this inactivity of certain siRNAs might be due to mRNA folding structure or blockage of cleavage sites by impenetrable protein coverage (10).

### Example 4 Analysis of the time-course and persistence of siRNA silencing

The time-course of mRNA silencing was measured, and Northern analysis of cells harvested 4, 8, 24 and 48 hours after start of transfection showed maximum siRNA silencing after 24 hours (Fig. 5a). There seemed to be a difference in the apparent depletion rate, as hTF167i reduced the mRNA level more than hTF173i at each time-point. Similar observations were made for modified versions of hTF 167i, in which the induced mutations (M1 and M2) resulted in reduced inhibitory activity. Mutations in the anti-sense strand had a more pronounced effect than the corresponding mutations in the sense strand. The fact that siRNA-induced target degradation was incomplete (a level of approximately 10% of the target mRNA remained even with the most effective siRNAs), may be due to the presence of a fraction of mRNA in a protected compartment, e.g. in spliceosomes or in other nuclear locations. However, in view of the above data and data from competition experiments, a more likely possibility may be a kinetically determined balance between transcription and degradation, the latter being a time-consuming process.

Experiments in plants (11) and nematodes (12, 13) have suggested the existence of a system whereby certain siRNA genes are involved in the heritability of induced phenotypes. To investigate the existence of such propagators in mammalian cell lines, the persistence of the siRNA silencing in HaCaT cells transfected at a very low cell density was measured. In an experiment based on serial transfection of reporter constructs there was a gradual recovery of expression between 3 and 5 days post-transfection, and the time-dependence of the siRNA effect on endogenous TF mRNA was similar (Fig. 5b). The level of TF mRNA in mock-transfected control cells declined gradually during the experiment, in what appeared to be cell expansion-dependent down-regulation of expression. Interestingly, the procoagulant activity showed little indication of recovering to control levels in transfected cells (Fig. 5b, columns). Similar observations were made with hTF372i and with a combination of hTF167i, hTF372i and hTF562i (data not shown).

### Example 5 Analysis of the effect of introducing base-pairing mutations in the siRNA sequences.

As mentioned previously, the present siRNA were mapped more systematically in order to determine whether mutations were equally tolerated within the whole siRNA. A total of 8 different new single-mutant siRNA were designed and named according to the position (starting from the 5' of the sense strand) of the mutation (s1, s2, s3, s4, s7, s11, s13, s16, i.e. SEQ ID NO 9- 17). The previously described central single-mutant M1 (eksempel 4) was included in this analysis and renamed s10. All siRNAs were analysed for productive annealing by denaturing PAGE (15%).

All the various mutant siRNAs were analysed for depletion of endogenous TF mRNA in HaCaT cells, 24h after LIPOFECTAMINE2000-mediated transfection, as previously described. A summary of the data is shown in figure 7. The wild type siRNA, and the mutant s10, included as positive controls, depleted TF mRNA to ca 10% and 20% residual levels, as expected and previously reported. The activities of the other mutants fall in three different groups depending on their position along the siRNA. Mutations in the extreme 5' end of the siRNA (s1-s3) were very well tolerated, exhibiting essentially the same activity as the wild type. Mutations located further in, up to the approximate midpoint of the siRNA (s4, s7, s10, s11), were slightly impaired in their activity, resulting in depletion of mRNA to 25-30% residual levels. Both the mutations in the 3' half of the siRNA, however, exhibited severely impaired activity. This suggested to us a bias in the tolerance for mutations in the siRNA. The activities of several double mutants, in which the central position (s10) was mutated in conjunction with one additional position (s7, s11, s13, s16), were also analysed. The bias in mutation tolerance was also evident for these double mutants, as the rank order of their activity mirrored that of the activity of the single mutants of the variant position. This observation strengthens the proposition that the differential activity of mutants is due to an intrinsic bias in the tolerance for target mismatches along the sequence of the siRNA. The reason for such a bias might be linked to the proposed existence of a ruler region in the siRNA which is primarily used by the RISC complex to "measure up" the target mRNA for cleavage (15).

### Example 6 Effects of chemical modification of the siRNA sequences.

A series of siRNAs with one modification each in the extreme 5' and 3' ends of the siRNA strands (P1+1, M1+1, A1+1, i.e. SEQ ID NO 22, 26 and 30, respectively) was initially synthesized. The 5' end of the chemically synthesized siRNAs might be more sensitive to modification since it has to be phosphorylated in vivo to be active (24). We therefore also included siRNAs with two modifications only in the non-base pairing 3' overhangs (siRNAs P0+2, M0+2 and A0+2, i.e. SEQ ID NO 23, 27 and 31, respectively, cf. figure 6), which were known to be tolerant for various types of modifications (10, 15, 25). Northern analysis of transfected HaCaT cells demonstrated essentially undiminished activity of all the modified siRNAs, with the exception of the siRNA with allylation at both ends (figure 8). Allyl-modification in the 3' end only had no effect on activity. The presence of a large substituent in 2'-hydroxyl of 5' terminal nucleotide might interfere with the proper phosphorylation of the siRNA shown to be necessary by Nykänen et al (24).

We next wanted to know if any of these modifications were sufficient to increase the persistence of siRNA-mediated silencing. Endogenous TF mRNA recovers gradually 3-5 days after transfection with wild type siRNA targeting hTF167. Transfecting HaCaT cells with active and chemically modified siRNA in parallel, we could not demonstrate any significant difference in the silencing activities 3 and 5 days post-transfection (data not shown). The moderate modifications we had introduced, although exhibiting full initial activity, were therefore clearly not sufficient to substantially stabilize the siRNAs in vivo.

With the activity of the siRNA still intact after our initial moderate modifications, the degree of modifications was extended to include either two on both sides or two on the 5' end in combination with four in the 3' end. Due to the less promising results with the allylated versions from the first series, and the higher cost associated with these modifications, we decided to restrict ourselves to phophorothioate modifications and methylations for the next series. The new set of siRNAs were likewise analysed for initial activity 24h following transfection into HaCaT cells. Normalized expression levels in cells transfected with modified siRNAs were slightly elevated, at 16-18% residual levels compared to 11% in cells transfected with wild type. The most extensively phosphorothioated siRNA proved to be toxic to cells, resulting in approximately 70% cell death compared to mock-transfected cells (measured as the expression level of the control mRNA GAPDH). Due to these complications, this siRNA species was not included in further analysis. The remaining siRNA species were evaluated for increased persistence of silencing by analysing TF mRNA expression 5 days after a single transfection of 100nM siRNA. At this point, TF expression in cells transfected with wild type siRNA had recovered almost completely (80% residual expression compared to mock-transfected cells) (figure 9a). In cells transfected with the most extensively modified siRNA (M2+4; SEQ ID NO 29), however, strong silencing was still evident (32% residual expression). The less extensively modified siRNA species (P2+2, M2+2; SEQ ID NO 24 and SEQ ID NO 28 respectively), although less effective than Me2+4, consistently resulted in lower TF expression 5 days post-transfection (55-60%) than the wild type. Time-course experiments demonstrated that the wild type siRNA was still the most effective 3 days post-transfection, when silencing was relatively unimpaired, but that silencing drops off at a much higher rate thereafter (figure 9b).

### REFERENCES

1. Bernstein E, Caudy AA, Hammond SM, Hannon GJ. Role for a bidentate ribonuclease in the initiation step of RNA interference. Nature 409, 363-366 (2001).
2. Camerer E, Kolsto AB, Prydz H. Cell biology of tissue factor, the principal initiator of blood coagulation. Thromb Res. 81, 1-41 (1996).
3. Clemens, J. C. et al. Use of double-stranded RNA interference in Drosophila cell lines to dissect signal transduction pathways. Proc. Natl Acad. Sci. USA 97, 6499-6503 (2000).
4. Amarzguioui M, Brede G, Babaie E, Grotli M, Sproat B, Prydz H. Secondary structure prediction and in vitro accessibility of mRNA as tools in the selection of target sites for ribozymes. Nucleic Acids Res. 28, 4113-4124 (2000).
5. Hutvagner G, McLachlan J, Pasquinelli AE, Balint E, Tuschl T, Zamore PD. A cellular function for the RNA-interference enzyme Dicer in the maturation of the let-7 small temporal RNA. Science 293, 834-838 (2001).
6. Wiiger MT, Pringle S, Pettersen KS, Narahara N, Prydz H. Effects of binding of ligand (FVIIa) to induced tissue factor in human endothelial cells. Thromb Res. 98, 311-321 (2000).
7. Camerer E, Pringle S, Skartlien AH, Wiiger M, Prydz K, Kolsto AB, Prydz H. Opposite sorting of tissue factor in human umbilical vein endothelial cells and Madin-Darby canine kidney epithelial cells. Blood. 88, 1339-1349 (1996).
8. Caplen, N. J., Fleenor, J., Fire, A. & Morgan, R. A. dsRNA-mediated gene silencing in cultured Drosophila cells: a tissue culture model for the analysis of RNA interference. Gene 252, 95-105 (2000).
9. Tuschl T, Zamore PD, Lehmann R, Bartel DP, Sharp PA. Targeted mRNA degradation by double-stranded RNA in vitro. Genes Dev. 13, 3191-3197 (1999).
10. Elbashir SM, Harborth J, Lendeckel W, Yalcin A, Weber K, Tuschl T. Duplexes of 21-nucleotide RNAs mediate RNA interference in cultured mammalian cells. Nature 411, 494-498 (2001).
11. Palauqui JC, Balzergue S. Activation of systemic acquired silencing by localised introduction of DNA. Curr Biol. 9, 59-66 (1999).
12. Fire, A. RNA-triggered gene silencing. Trends Genet. 15, 358-363 (1999)
13. Grishok A, Tabara H, Mello CC. Genetic requirements for inheritance of RNAi in C. elegans. Science 287, 2494-2497 (2000).
14. Boutla, A., Delidakis, C., Livadaras, L, Tsagris, M. and Tabler, M. (2001) Short 5'-phosphorylated double-stranded RNAs induce RNA interference in Drosophila. Curr. Biol., 11:1776-1780.
15. Elbashir, S.M., Martinez, J., Patkaniowska, A., Lendeckel, W. and Tuschl, T. (2001) Functional anatomy of siRNAs for mediating effiecient RNAi in Drosophila melanogaster embryo lysate. EMBO J., 20:6877-6888.
16. Jacque, J.M., Triques, K., Stevenson, M. (2002) Modulation of HIV-1 replication by RNA interference. Nature, 418: 435-438.
17. Gitlin, L., Karelsky, S., Andino, R. (2002) Short interfering RNA confers intracellular antiviral immunity in human cells. Nature, 418:430-434.
18. Klahre, U., Crete, P., Leuenberger, S.A., Iglesias, V.A., Meins, F. Jr. (2002) High molecular weight RNAs and small interfering RNAs induce systemic posttranscriptional gene silencing in plants. Proc. Natl. Acad. Sci. USA. 99:11981-11986.
19. Garrus, J.E., von Schwedler, U.K., Pornillos, O.W., Morham, S.G., Zavitz, K.H., Wang, H.E., Wettstein, D.A., Stray, K.M., Cote, M., Rich, R.L., Myszka, D.G., Sundquist, W.I. (2001). Tsg101 and the vacuolar protein sorting pathway are essential for HIV-1 budding. Cell, 107:55-65.
20. Yu, J.Y., DeRuiter, S.L., Turner, D.L. (2002). RNA interference by expression of short-interfering RNAs and hairpin RNAs in mammalian cells. Proc. Natl. Acad. Sci. USA, 99:6047-52.
21. Wilda, M., Fuchs, U., Wossmann, W., Borkhardt, A. (2002). Killing of leukemic cells with a BCR/ABL fusion gene by RNA interference (RNAi). Oncogene, 21:5716-24.
22. Brummelkamp, T.R., Bernards, R., Agami, R. (2002) A system for stable expression of short interfering RNAs in mammalian cells. Science, 296:550-3.
23. Paddison, P.J., Caudy, A.A., Bernstein, E., Hannon, G.J., Conklin, D.S. (2002) Short hairpin RNAs (shRNAs) induce sequence-specific silencing in mammalian cells. Genes Dev., 16:948-58.
24. Nykänen, A., Haley, B. and Zamore, P.D. (2001) ATP Requirements and Small Interfering RNA Structure in the RNA Interference Pathway. Cell, 107:309-321.
25. Elbashir, S.M., Lendeckel, W. and Tuschl, T. (2001) RNA interference is mediated by 21 and 22 nt RNAs. Genes Dev., 15:188-200.
26. Janowsky, E., and Schwenzer, B. (1998), Oligonucleotide facilitators enable a hammerhead ribozyme to cleave long RNA substrates with multiple-turnover activity. Eur. J. Biochem., 254, 129-134.
27. Billy, E. et al. (2001),Specific interference with gene expression induced by long, double-stranded RNA in mouse embryonal teratocarcinoma cell lines., Proc. Natl. Acad. Sci. USA, 98:25, 14428-33.
28. Caplen, N.J. et al. (2001), Specific inhibition of gene expression by small double-stranded RNAs in invertebrate and vertebrate systems., Proc. Natl. Acad. Sci. USA, 98:17, 9742-47.
29. Holen, T. et al. (2002), Positional effects of short interfering RNAs targeting the human coagulation trigger Tissue Factor, Nucleic Acid Res., 30, 1757-1766.
30. Tuschl, T. and Borkhardt, A. (2002), Small interfering RNAs: a revolutionary tool for the analyses of gene function and gene therapy, Mol. Interv., 2, 158-167.
31. Harborth et al. (2001), Identification of essential genes in cultured mammalian cells using small interfering RNAs, J. Cell Science, 114, 4557-4565.

## Claims

1. Short interfering RNA molecule (siRNA), **characterized in** comprising at least 19 nucleotides directed towards a tissue factor (TF) coding nucleic acid sequence or fragments thereof, and wherein the siRNA molecule comprise a nucleotide sequence selected from the group consisting of
**hTF167** 
5′ g-c-g-c-u-u-c-a-g-g-c-a-c-u-a-c-a-a-a-t-t
t-t-c-g-c-g-a-a-g-u-c-c-g-u-g-a-u-g-u-u-u 5′;
 **hTF372** 
5′ g-a-a-g-c-a-g-a-c-g-u-a-c-u-u-g-g-c-a-t-t
t-t-c-u-u-c-g-u-c-u-g-c-a-u-g-a-a-c-c-g-u 5′;
 **hTF562** 
5' c-g-g-a-c-u-u-u-a-g-u-c-a-g-a-a-g-g-a-t-t
t-t-g-c-c-u-g-a-a-a-u-c-a-g-u-c-u-u-c-c-u 5';
 **hTF256** 
5' c-c-c-g-u-c-a-a-u-c-a-a-g-u-c-u-a-c-a-t-t
t-t-g-g-g-c-a-g-u-u-a-g-u-u-c-a-g-a-u-g-u 5';
 **hTF164** 
5' c-c-g-g-c-g-c-u-u-c-a-g-g-c-a-c-u-a-c-t-t
t-t-g-g-c-c-g-c-g-a-a-g-u-c-c-g-u-g-a-u-g 5';
 **hTF170** 
5' c-u-u-c-a-g-g-c-a-c-u-a-c-a-a-a-u-a-c-t-t
t-t-g-a-a-g-u-c-c-g-u-g-a-u-g-u-u-u-a-u-g 5';
 **hTF173** 5' c-a-g-g-c-a-c-u-a-c-a-a-a-u-a-c-u-g-u-t-t t-t-g-u-c-c-g-u-g-a-u-g-u-u-u-a-u-g-a-c-a  5'; **hTF167-s2** 
5′ g-g-g-c-u-u-c-a-g-g-c-a-c-u-a-c-a-a-a-u-a
g-c-c-c-g-a-a-g-u-c-c-g-u-g-a-u-g-u-u-u 5′;
 **hTF167-s3**

5′ g-c-c-c-u-u-c-a-g-g-c-a-c-u-a-c-a-a-a-u-a
g-c-c-g-g-g-a-a-g-u-c-c-g-u-g-a-u-g-u-u-u 5′.

2. RNA molecule (siRNA) according to claim 1, **characterized in that** said molecule is double stranded.

3. RNA molecule (siRNA) according to claims 1-2, **characterized in that** said molecule is 21-25 nucleotides long, preferably 21 nucleotides long.

4. RNA molecule (siRNA) according to any of claims 1-3, **characterized in that** said molecule consists of one of the sequences selected from the group consisting of
**hTF167** 
5′ g-c-g-c-u-u-c-a-g-g-c-a-c-u-a-c-a-a-a-t-t
t-t-c-g-c-g-a-a-g-u-c-c-g-u-g-a-u-g-u-u-u 5′;
 **hTF372** 
5′ g-a-a-g-c-a-g-a-c-g-u-a-c-u-u-g-g-c-a-t-t
t-t-c-u-u-c-g-u-c-u-g-c-a-u-g-a-a-c-c-g-u 5′;
 **hTF562** 
5′  c-g-g-a-c-u-u-u-a-g-u-c-a-g-a-a-g-g-a-t-t
t-t-g-c-c-u-g-a-a-a-u-c-a-g-u-c-u-u-c-c-u  5′;
 **hTF256** 
5′ c-c-c-g-u-c-a-a-u-c-a-a-g-u-c-u-a-c-a-t-t
t-t-g-g-g-c-a-g-u-u-a-g-u-u-c-a-g-a-u-g-u 5′;
 **hTF164** 
5' c-c-g-g-c-g-c-u-u-c-a-g-g-c-a-c-u-a-c-t-t
t-t-g-g-c-c-g-c-g-a-a-g-u-c-c-g-u-g-a-u-g 5';
 **hTF170** 
5′  c-u-u-c-a-g-g-c-a-c-u-a-c-a-a-a-u-a-c-t-t
t-t-g-a-a-g-u-c-c-g-u-g-a-u-g-u-u-u-a-u-g  5′;
 **hTF173**

5′ c-a-g-g-c-a-c-u-a-c-a-a-a-u-a-c-u-g-u-t-t
t-t-g-u-c-c-g-u-g-a-u-g-u-u-u-a-u-g-a-c-a 5′.

5. RNA molecule (siRNA) according to any of claims 1-3, **characterized in that** one or more mutations are introduced in relation to the wild type sequence wherein said molecule consists of the sequence
**hTF167-s2** 
5' g-g-g-c-u-u-c-a-g-g-c-a-c-u-a-c-a-a-a-u-a
g-c-c-c-g-a-a-g-u-c-c-g-u-g-a-u-g-u-u-u 5'; or
 **hTF167-s3**

5′ g-c-c-c-u-u-c-a-g-g-c-a-c-u-a-c-a-a-a-u-a
g-c-c-g-g-a-a-g-u-c-c-g-u-g-a-u-g-u-u-u 5′.

6. RNA molecule (siRNA) according to claim 1, **characterized in that** said siRNA molecule is modified by replacing the phosphodiester bond with a thiophosphodiester bond.

7. RNA molecule (siRNA) according to claim 6, **characterized in that** said molecule is selected from the group consisting of
**hTF167-P1+1** 
5′  g*c-g-c-u-u-c-a-g-g-c-a-c-u-a-c-a-a-a-u*a
g*c-c-g-c-g-a-a-g-u-c-c-g-u-g-a-u-g-u-u*u  5′
 **hTF167-P0+2** 
5' g-c-g-c-u-u-c-a-g-g-c-a-c-u-a-c-a-a-a*u*a
g*c*c-g-c-g-a-a-g-u-c-c-g-u-g-a-u-g-u-u-u 5'
 **hTF167-P2+2** 
5'  g*c*g-c-u-u-c-a-g-g-c-a-c-u-a-c-a-a-a*u*a
g*c*c-g-c-g-a-a-g-u-c-c-g-u-g-a-u-g-u*u*u  5'
 **hTF167-P2+4**
wherein the asterisk indicates nucleotides joined by phosphorothioate bonds.

5′  g*c*g-c-u-u-c-a-g-g-c-a-c-u-a-c-a*a*a*u*a
g*c*c*g*c-g-a-a-g-u-c-c-g-u-g-a-u-g-u*u*u  5′.

8. RNA molecule (siRNA) according to claim 7, **characterized in that** said molecule is
**hTF167-P2+2** 
5' g*c*g-c-u-u-c-a-g-g-c-a-c-u-a-c-a-a-a*u*a
g*c*c-g-c-g-a-a-g-u-c-c-g-u-g-a-u-g-u*u*u 5',
 wherein the asterisk indicates nucleotides joined by phosphorothioate bonds.

9. RNA molecule (siRNA) according to any of claims 1-3, **characterized in that** said molecule is modified by the introduction of a C₁-C₃-alkyl, C₁-C₃-alkenyl or C₁-C₃-alkylyl group in one or more of the 2' OH hydroxyl groups in the sequence.

10. RNA molecule (siRNA) according to claim 9, **characterized in that** said molecule is selected from a group consisting of
**hTF167-M1+1** 
5' G-c-g-c-u-u-c-a-g-g-c-a-c-u-a-c-a-a-a-u-A
G-c-c-g-c-g-a-a-g-u-c-c-g-u-g-a-u-g-u-u-U 5'
 **hTF167-M0+2** 
5′ g-c-g-c-u-u-c-a-g-g-c-a-c-u-a-c-a-a-a-U-A
G-C-c-g-c-g-a-a-g-u-c-c-g-u-g-a-u-g-u-u-u 5′
 **hTF167-M2+2** 
5' G-C-g-c-u-u-c-a-g-g-c-a-c-u-a-c-a-a-a-U-A
G-C-c-g-c-g-a-a-g-u-c-c-g-u-g-a-u-g-u-U-U 5'
 **hTF167-M2+4** 
5' G-C-g-c-u-u-c-a-g-g-c-a-c-u-a-c-a-A-A-U-A
G-C-C-G-c-g-a-a-g-u-c-c-g-u-g-a-u-g-u-U-U 5'
 **hTF167-A1+1** 
5′ G-c-g-c-u-u-c-a-g-g-c-a-c-u-a-c-a-a-a-u-A
G-c-c-g-c-g-a-a-g-u-c-c-g-u-g-a-u-g-u-u-U 5′
 **hTF167-A0+2**
wherein a bold capital letter indicates that the nucleotide is 2-O-methylated and wherein an underlined bold capital letter indicates that the nucleotide is 2-O-allylated.

5' g-c-g-c-u-u-c-a-g-g-c-a-c-u-a-c-a-a-a-U-A
G-C-c-g-c-g-a-a-g-u-c-c-g-u-g-a-u-g-u-u-u 5',

11. RNA molecule (siRNA) according to claim 10, **characterized in that** said molecule consists of the sequence
**hTF167-M2+2** 
5' G-C-g-c-u-u-c-a-g-g-c-a-c-u-a-c-a-a-a-U-A
G-C-c-g-c-g-a-a-g-u-c-c-g-u-g-a-u-g-u-U-U 5' or
 **hTF167-M2+4** 
5' G-C-g-c-u-u-c-a-g-g-c-a-c-u-a-c-a-A-A-U-A
G-C-C-G-c-g-a-a-g-u-c-c-g-u-g-a-u-g-u-U-U 5',
 wherein a bold capital letter indicates that the nucleotide is 2-O-methylated.

12. RNA molecule (siRNA) according to claim 10, **characterized in that** said molecule consists of the sequence
**hTF167-M2+4** 
5′ G-C-g-c-u-u-c-a-g-g-c-a-c-u-a-c-a-A-A-U-A
G-C-C-G-c-g-a-a-g-u-c-c-g-u-g-a-u-g-u-U-U 5′,
 wherein a bold capital letter indicates that the nucleotide is 2-O-methylated.

13. RNA molecule (siRNA) according to any of claims 1-11, **characterized in that** the TF or fragment thereof is of vertebrate origin, preferably mammalian origin, more preferably human origin.

14. Pharmaceutical preparation, **characterized in that** it comprises one or more of the short interfering RNA molecules (siRNA) according to any of claims 1-13.

15. Pharmaceutical preparation according to claim 14
**characterized in that** it comprises the sequence
**hTF167** 
5′ g-c-g-c-u-u-c-a-g-g-c-a-c-u-a-c-a-a-t-t
t-t-c-g-c-g-a-a-g-u-c-c-g-u-g-a-u-g-u-u 5′;
 or
**hTF372**

5′ g-a-a-g-c-a-g-a-c-g-u-a-c-u-u-g-g-c-a-t-t
t-t-c-u-u-c-g-u-c-u-g-c-a-u-g-a-a-c-c-g-u 5′.

16. Pharmaceutical preparation according to claim 14, **characterized in that** it comprises the sequence
**hTF167-s2** 
5' g-g-g-c-u-u-c-a-g-g-c-a-c-u-a-c-a-a-a-u-a
g-c-c-c-g-a-a-g-u-c-c-g-u-g-a-u-g-u-u-u 5';
 or
**hTF167-s3**

5′ g-c-c-c-u-u-c-a-g-g-c-a-c-u-a-c-a-a-a-u-a
g-c-c-g-g-g-a-a-g-u-c-c-g-u-g-a-u-g-u-u-u 5′.

17. Pharmaceutical preparation according to claim 14, **characterized in that** said molecules are selected from the group consisting of:
**hTF167-P2+2** 
5' g*c*g-c-u-u-c-a-g-g-c-a-c-u-a-c-a-a-a*u*a
g*c*c-g-c-g-a-a-g-u-c-c-g-u-g-a-u-g-u*u*u 5';
 **hTF167-M2+2** 
5′ G-C-g-c-u-u-c-a-g-g-c-a-c-u-a-c-a-a-a-U-A
G-C-c-g-c-g-a-a-g-u-c-c-g-u-g-a-u-g-u-U-U 5′;
 or
**hTF167-M2+4** 
5' G-C-g-c-u-u-c-a-g-g-c-a-c-u-a-c-a-A-A-U-A
G-C-C-G-c-g-a-a-g-u-c-c-g-u-g-a-u-g-u-U-U 5',
 wherein the asterisk indicates nucleotides joined by phosphorothioate bonds and wherein a bold capital letter indicates that the nucleotide is 2-O-methylated.

18. Pharmaceutical preparation according to claim 17 wherein the RNA molecule is an isolated RNA molecule comprising the sequence
**hTF167-M2+4** 
5' G-C-g-c-u-u-c-a-g-g-c-a-c-u-a-c-a-A-A-U-A
G-C-C-G-c-g-a-a-g-u-c-c-g-u-g-a-u-g-u-U-U 5',
 wherein a bold capital letter indicates that the nucleotide is 2-O-methylated.

19. Pharmaceutical preparation according to any of the claims 14 - 18, **characterised in that** it furthermore comprises diluents, lubricants, binders, carriers, disintegration means, absorption means, colourings, sweeteners and/or flavourings.

20. Pharmaceutical preparation according to any of claims 14-19, **characterized in that** it comprises adjuvants.

21. Pharmaceutical preparation according to any of claims 14-20, suitable to be administered parenterally, preferably by subcutaneous, intravenous, intramuscular or intraperitonal injection or infusion, orally, nasally, buccally, rectally, vaginally and/or by inhalation or insufflation.

22. Pharmaceutical preparation according to any of claims 14-21, **characterized in that** it is formulated as infusion solutions or suspensions, an aerosol, capsules, tablets, pills, spray, suppositories, in dosage formulations containing conventional non-toxic pharmaceutically-acceptable carriers, adjuvants and/or vehicles.

23. The use of one or more of the RNA molecules (siRNA) according to any of the claims 1-13 to prepare a pharmaceutical preparation suitable for the treatment, prevention and/or inhibition of unwanted blood coagulation.

24. The use according to claim 23, wherein said preparation is formulated for parenteral administration, preferably subcutaneous, intravenous, intramuscular or intraperitonal injection or infusion, oral, nasal, buccal, rectal, vaginal administration and/or by inhalation or insufflation.

## Patentansprüche

1. short interfering RNA-Molekül (siRNA), **dadurch gekennzeichnet, dass** es mindestens 19 Nukleotide umfasst, die gegen eine Nukleinsäuresequenz, die für den Tissue Faktor (TF) oder ein Fragment davon kodiert, gerichtet ist, und worin das siRNA-Molekül eine Nukleotidsequenz umfasst, ausgewählt aus der Gruppe bestehend aus
**hTF167** 
5' g-c-g-c-u-u-c-a-g-g-c-a-c-u-a-c-a-a-a-t-t
t-t-c-g-c-g-a-a-g-u-c-c-g-u-g-a-u-g-u-u-u 5';
 **hTF372** 
5′ g-a-a-g-c-a-g-a-c-g-u-a-c-u-u-g-g-c-a-t-t
t-t-c-u-u-c-g-u-c-u-g-c-a-u-g-a-a-c-c-g-u 5′;
 **hTF562** 
5' c-g-g-a-c-u-u-u-a-g-u-c-a-g-a-a-g-g-a-t-t
t-t-g-c-c-u-g-a-a-a-u-c-a-g-u-c-u-u-c-c-u 5';
 **hTF256** 
5′  c-c-c-g-u-c-a-a-u-c-a-a-g-u-c-u-a-c-a-t-t
t-t-g-g-g-c-a-g-u-u-a-g-u-u-c-a-g-a-u-g-u  5′;
 **hTF164** 
5' c-c-g-g-c-g-c-u-u-c-a-g-g-c-a-c-u-a-c-t-t
t-t-g-g-c-c-g-c-g-a-a-g-u-c-c-g-u-g-a-u-g 5';
 **hTF170** 
5′  c-u-u-c-a-g-g-c-a-c-u-a-c-a-a-a-u-a-c-t-t
t-t-g-a-a-g-u-c-c-g-u-g-a-u-g-u-u-u-a-u-g  5′;
 **hTF173** 5′ c-a-g-g-c-a-c-u-a-c-a-a-a-u-a-c-u-g-u-t-t t-t-g-u-c-c-g-u-g-a-u-g-u-u-u-a-u-g-a-c-a  5'; **hTF167-s2** 
5' g-g-g-c-u-u-c-a-g-g-c-a-c-u-a-c-a-a-a-u-a
g-c-c-c-c-g-a-a-g-u-c-c-g-u-g-a-u-g-u-u-u 5';
 **hTF167-s3**

5′ g-c-c-c-u-u-c-a-g-g-c-a-c-u-a-c-a-a-a-u-a
g-c-c-g-g-g-a-a-g-u-c-c-g-u-g-a-u-g-u-u-u 5′.

2. RNA-Molekül (siRNA) nach Anspruch 1, **dadurch gekennzeichnet, dass** das Molekül doppel-strängig ist.

3. RNA-Molekül (siRNA) nach den Ansprüchen 1-2, **dadurch gekennzeichnet, dass** das Molekül 21-25 Nukleotide lang, bevorzugt 21 Nukleotide lang ist.

4. RNA-Molekül (siRNA) nach einem der Ansprüche 1-3, **dadurch gekennzeichnet, dass** das Molekül aus einer der Sequenzen, ausgewählt aus der Gruppe bestehend aus
**hTF167** 
5′ g-c-g-c-u-u-c-a-g-g-c-a-c-u-a-c-a-a-a-t-t
t-t-c-g-c-g-a-a-g-u-c-c-g-u-g-a-u-g-u-u-u 5′;
 **hTF372** 
5' g-a-a-g-c-a-g-a-c-g-u-a-c-u-u-g-g-c-a-t-t
t-t-c-u-u-c-g-u-c-u-g-c-a-u-g-a-a-c-c-g-u 5';
 **hTF562** 
5' c-g-g-a-c-u-u-u-a-g-u-c-a-g-a-a-g-g-a-t-t
t-t-g-c-c-u-g-a-a-a-u-c-a-g-u-c-u-u-c-c-u 5';
 **hTF256** 
5′ c-c-c-g-u-c-a-a-u-c-a-a-g-u-c-u-a-c-a-t-t
t-t-g-g-g-c-a-g-u-u-a-g-u-u-c-a-g-a-u-g-u 5′;
 **hTF164** 
5′ c-c-g-g-c-g-c-u-u-c-a-g-g-c-a-c-u-a-c-t-t
t-t-g-g-c-c-g-c-g-a-a-g-u-c-c-g-u-g-a-u-g 5′;
 **hTF170** 
5′  c-u-u-c-a-g-g-c-a-c-u-a-c-a-a-a-u-a-c-t-t
t-t-g-a-a-g-u-c-c-g-u-g-a-u-g-u-u-u-a-u-g  5′;
 **hTF173** 
5' c-a-g-g-c-a-c-u-a-c-a-a-a-u-a-c-u-g-u-t-t
t-t-g-u-c-c-g-u-g-a-u-g-u-u-u-a-u-g-a-c-a 5'
 besteht.

5. RNA-Molekül (siRNA) nach einem der Ansprüche 1-3, **dadurch gekennzeichnet, dass** eine oder mehrere Mutation(en) in Bezug auf die Wildtypsequenz eingeführt wird/werden, worin das Molekül aus der Sequenz
**hTF167-s2** 
5′ g-g-g-c-u-u-c-a-g-g-c-a-c-u-a-c-a-a-a-u-a
g-c-c-c-c-g-a-a-g-u-c-c-g-u-g-a-u-g-u-u-u 5′;
 oder **hTF167-s3** 
5' g-c-c-c-u-u-c-a-g-g-c-a-c-u-a-c-a-a-a-u-a
g-c-c-g-g-g-a-a-g-u-c-c-g-u-g-a-u-g-u-u-u 5'
 besteht.

6. RNA-Molekül (siRNA) nach Anspruch 1, **dadurch gekennzeichnet, dass** das siRNA-Molekül modifiziert ist, indem die Phosphodiester-Bindung mit einer Thiophosphodiester-Bindung ersetzt wird.

7. RNA-Molekül (siRNA) nach Anspruch 6, **dadurch gekennzeichnet, dass** das Molekül ausgewählt ist aus der Gruppe bestehend aus
**hTF167-P1+1** 
5' g*c-g-c-u-u-c-a-g-g-c-a-c-u-a-c-a-a-a-u*a
g*c-c-g-c-g-a-a-g-u-c-c-g-u-g-a-u-g-u-u*u 5'
 **hTF167-P0+2** 
5′  g-c-g-c-u-u-c-a-g-g-c-a-c-u-a-c-a-a-a*u*a
g*c*c-g-c-g-a-a-g-u-c-c-g-u-g-a-u-g-u-u-u  5′
 **hTF167-P2+2** 
5′  g*c*g-c-u-u-c-a-g-g-c-a-c-u-a-c-a-a-a*u*a
g*c*c-g-c-g-a-a-g-u-c-c-g-u-g-a-u-g-u*u*u  5′
 **hTF167-P2+4** 
5′ g*c*g-c-u-u-c-a-g-g-c-a-c-u-a-c-a*a*a*u*a
g*c*c*g*c-g-a-a-g-u-c-c-g-u-g-a-u-g-u*u*u 5′,
 worin der Stern Nukleotide anzeigt, die durch Phosphorthioat-Bindungen verbunden sind.

8. RNA-Molekül (siRNA) nach Anspruch 7, **dadurch gekennzeichnet, dass** das Molekül
**hTF167-P2+2** 
5′  g*c*g-c-u-u-c-a-g-g-c-a-c-u-a-c-a-a-a*u*a
g*c*c-g-c-g-a-a-g-u-c-c-g-u-g-a-u-g-u*u*u  5′
 ist, worin der Stern Nukleotide anzeigt, die durch Phosphorthioat-Bindungen verbunden sind.

9. RNA-Molekül (siRNA) nach einem der Ansprüche 1-3, **dadurch gekennzeichnet, dass** das Molekül durch die Einführung einer C₁-C₃-Alkyl-, C₁-C₃-Alkenyl-oder C₁-C₃-Alkylyl-Gruppe in einer oder mehrerer der 2' OH-Hydroxylgruppen in der Sequenz modifiziert ist.

10. RNA-Molekül (siRNA) nach Anspruch 9, **dadurch gekennzeichnet, dass** das Molekül ausgewählt ist aus der Gruppe bestehend aus
**hTF167-M1+1** 
5' G-c-g-c-u-u-c-a-g-g-c-a-c-u-a-c-a-a-a-u-A
G-c-c-g-c-g-a-a-g-u-c-c-g-u-g-a-u-g-u-u-U 5'
 **hTF167-M0+2** 
5′ g-c-g-c-u-u-c-a-g-g-c-a-c-u-a-c-a-a-a-U-A
G-C-c-g-c-g-a-a-g-u-c-c-g-u-g-a-u-g-u-u-u 5′
 **hTF167-M2+2** 
5' G-C-g-c-u-u-c-a-g-g-c-a-c-u-a-c-a-a-a-U-A
G-C-c-g-c-g-a-a-g-u-c-c-g-u-g-a-u-g-u-U-U 5'
 **hTF167-M2+4** 
5' G-C-g-c-u-u-c-a-g-g-c-a-c-u-a-c-a-A-A-U-A
G-C-C-G-c-g-a-a-g-u-c-c-g-u-g-a-u-g-u-U-U 5'
 **hTF167-A1+1** 
5′ G-c-g-c-u-u-c-a-g-g-c-a-c-u-a-c-a-a-a-u-A
G-c-c-g-c-g-a-a-g-u-c-c-g-u-g-a-u-g-u-u-U 5′
 **hTF167-A0+2** 
5′ g-c-g-c-u-u-c-a-g-g-c-a-c-u-a-c-a-a-a-U-A
G-C-c-g-c-g-a-a-g-u-c-c-g-u-g-a-u-g-u-u-u 5′,
 worin ein fettgedruckter Großbuchstabe anzeigt, dass das Nukleotid 2-O-methyliert ist und worin ein unterstrichener, fettgedruckter Großbuchstabe anzeigt, dass das Nukleotid 2-O-allyliert ist.

11. RNA-Molekül (siRNA) nach Anspruch 10, **dadurch gekennzeichnet, dass** das Molekül aus der Sequenz
**hTF167-M2+2** 
5' G-C-g-c-u-u-c-a-g-g-c-a-c-u-a-c-a-a-a-U-A
G-C-c-g-c-g-a-a-g-u-c-c-g-u-g-a-u-g-u-U-U 5'
 oder
**hTF167-M2+4** 
5' G-C-g-c-u-u-c-a-g-g-c-a-c-u-a-c-a-A-A-U-A
G-C-C-G-c-g-a-a-g-u-c-c-g-u-g-a-u-g-u-U-U 5'
 besteht,
worin ein fettgedruckter Großbuchstabe anzeigt, dass das Nukleotid 2-O-methyliert ist.

12. RNA-Molekül (siRNA) nach Anspruch 10, **dadurch gekennzeichnet, dass** das Molekül aus der Sequenz
**hTF167-M2+4** 
5′  G-C-g-c-u-u-c-a-g-g-c-a-c-u-a-c-a-A-A-U-A
G-C-C-G-c-g-a-a-g-u-c-c-g-u-g-a-u-g-u-U-U  5′
 besteht,
worin ein fettgedruckter Großbuchstabe anzeigt, dass das Nukleotid 2-O-methyliert ist.

13. RNA-Molekül (siRNAs) nach einem der Ansprüche 1-11, **dadurch gekennzeichnet, dass** der TF oder das Fragment davon vertebraten Ursprungs ist, bevorzugt Säugetier-Ursprungs, stärker bevorzugt menschlichen Ursprungs.

14. Pharmazeutische Zubereitung, **dadurch gekennzeichnet, dass** es eines oder mehrere der short interfering RNA-Moleküle (siRNA) nach einem der Ansprüche 1-13 umfasst.

15. Pharmazeutische Zubereitung nach Anspruch 14, **dadurch gekennzeichnet, dass** es die Sequenz
**hTF167** 
5′ g-c-g-c-u-u-c-a-g-g-c-a-c-u-a-c-a-a-a-t-t
t-t-c-g-c-g-a-a-g-u-c-c-g-u-g-a-u-g-u-u-u 5′;
 oder
**hTF372** 
5′ g-a-a-g-c-a-g-a-c-g-u-a-c-u-u-g-g-c-a-t-t
t-t-c-u-u-c-g-u-c-u-g-c-a-u-g-a-a-c-c-g-u 5′
 umfasst.

16. Pharmazeutische Zubereitung nach Anspruch 14, **dadurch gekennzeichnet, dass** es die Sequenz
**hTF167-s2** 
5' g-g-g-c-u-u-c-a-g-g-c-a-c-u-a-c-a-a-a-u-a
g-c-c-c-c-g-a-a-g-u-c-c-g-u-g-a-u-g-u-u-u 5';
 oder
**hTF167-s3** 
5′  g-c-c-c-u-u-c-a-g-g-c-a-c-u-a-c-a-a-a-u-a
g-c-c-g-g-g-a-a-g-u-c-c-g-u-g-a-u-g-u-u-u  5′
 umfasst.

17. Pharmazeutische Zubereitung nach Anspruch 14, **dadurch gekennzeichnet, dass** die Moleküle ausgewählt sind aus der Gruppe bestehend aus:
**hTF167-P2+2** 
5' g*c*g-c-u-u-c-a-g-g-c-a-c-u-a-c-a-a-a*u*a
g*c*c-g-c-g-a-a-g-u-c-c-g-u-g-a-u-g-u*u*u 5';
 **hTF167-M2+2** 
5' G-C-g-c-u-u-c-a-g-g-c-a-c-u-a-c-a-a-a-U-A
G-C-c-g-c-g-a-a-g-u-c-c-g-u-g-a-u-g-u-U-U 5';
 oder
**hTF167-M2+4** 
5′ G-C-g-c-u-u-c-a-g-g-c-a-c-u-a-c-a-A-A-U-A
G-C-C-G-c-g-a-a-g-u-c-c-g-u-g-a-u-g-u-U-U 5′,
 worin der Stern Nukleotide anzeigt, die durch Phosphorthioat-Bindungen verbunden sind und worin ein fettgedruckter Großbuchstabe anzeigt, dass das Nukleotid 2-O-methyliert ist.

18. Pharmazeutische Zubereitung nach Anspruch 17, worin das RNA-Molekül ein isoliertes RNA-Molekül ist, das die Sequenz
**hTF167-M2+4** 
5' G-C-g-c-u-u-c-a-g-g-c-a-c-u-a-c-a-A-A-U-A
G-C-C-G-c-g-a-a-g-u-c-c-g-u-g-a-u-g-u-U-U 5'
 umfasst, worin ein fettgedruckter Großbuchstabe anzeigt, dass das Nukleotid 2-O-methyliert ist.

19. Pharmazeutische Zubereitung nach einem der Ansprüche 14-18, **dadurch gekennzeichnet, dass** es weiterhin Verdünnungsmittel, Gleitmittel, Bindemittel, Träger, Sprengmittel, Absorptionsmittel, Färbemittel, Süßungsmittel und/oder Aromastoffe
umfasst.

20. Pharmazeutische Zubereitung nach einem der Ansprüche 14-19, **dadurch gekennzeichnet, dass** es Hilfsstoffe umfasst.

21. Pharmazeutische Zubereitung nach einem der Ansprüche 14-20, die dazu geeignet ist parenteral verabreicht zu werden, bevorzugt durch subkutane, intravenöse, intramuskuläre oder intraperitoneale Injektion oder Infusion, oral, nasal, bukkal, rektal, vaginal und/oder durch Inhalation oder Insufflation.

22. Pharmazeutische Zubereitung nach einem der Ansprüche 14-21, **dadurch gekennzeichnet, dass** sie als Infusionslösungen oder Suspensionen, ein Aerosol, Kapseln, Tabletten, Pillen, Spray, Zäpfchen formuliert ist, in Dosierungformulierungen, die herkömmliche nicht-toxische pharmazeutisch akzeptable Träger, Hilfsstoffe und/oder Vehikel enthalten.

23. Verwendung eines oder mehrerer der RNA-Moleküle (siRNA) nach einem der Ansprüche 1-13, um eine pharmazeutische Zubereitung herzustellen, die für die Behandlung, Prävention und/oder Inhibition unerwünschter Blutgerinnung geeignet ist.

24. Verwendung nach Anspruch 23, worin die Zubereitung für eine parenterale Verabreichung formuliert ist, bevorzugt subkutane, intravenöse, intramuskuläre oder intraperitoneale Injektion oder Infusion, orale, nasale, bukkale, rektale, vaginale Verabreichung und/oder durch Inhalation oder Insufflation.

## Revendications

1. Molécule d'ARN interférent court (siARN), **caractérisée en ce qu'**elle comprend au moins 19 nucléotides dirigés vers une séquence d'acide nucléique codant pour le facteur tissulaire (TF) ou ses fragments, ladite molécule de siARN comprenant une séquence de nucléotides choisie dans le groupe consistant en :
**hTF167** 
5' g-c-g-c-u-u-c-a-g-g-c-a-c-u-a-c-a-a-a-t-t
t-t-c-g-c-g-a-a-g-u-c-c-g-u-g-a-u-g-u-u-u 5' ;
 **hTF372** 
5' g-a-a-g-c-a-g-a-c-g-u-a-c-u-u-g-g-c-a-t-t
t-t-c-u-u-c-g-u-c-u-g-c-a-u-g-a-a-c-c-g-u 5' ;
 **htf562** 
5' c-g-g-a-c-u-u-u-a-g-u-c-a-g-a-a-g-g-a-t-t
t-t-g-c-c-u-g-a-a-a-u-c-a-g-u-c-u-u-c-c-u 5' ;
 **hTF256** 
5' c-c-c-g-u-c-a-a-u-c-a-a-g-u-c-u-a-c-a-t-t
t-t-g-g-g-c-a-g-u-u-a-g-u-u-c-a-g-a-u-g-u 5' ;
 **hTF164** 
5' c-c-g-g-c-g-c-u-u-c-a-g-g-c-a-c-u-a-c-t-t
t-t-g-g-c-c-g-c-g-a-a-g-u-c-c-g-u-g-a-u-g 5' ;
 **hTF170** 
5' c-u-u-c-a-g-g-c-a-c-u-a-c-a-a-a-u-a-c-t-t
t-t-g-a-a-g-u-c-c-g-u-g-a-u-g-u-u-u-a-u-g 5' ;
 **hTF173** 
5' c-a-g-g-c-a-c-u-a-c-a-a-a-u-a-c-u-g-u-t-t
t-t-g-u-c-c-g-u-g-a-u-g-u-u-u-a-u-g-a-c-a 5' ;
 **hTF167-s2** 
5' g-g-g-c-u-u-c-a-g-g-c-a-c-u-a-c-a-a-a-u-a
g-c-c-c-c-g-a-a-g-u-c-c-g-u-g-a-u-g-u-u-u 5' ;
 **htf167-s3**

5' g-c-c-c-u-u-c-a-g-g-c-a-c-u-a-c-a-a-a-u-a
g-c-c-g-g-g-a-a-g-u-c-c-g-u-g-a-u-g-u-u-u 5'.

2. Molécule d'ARN (siARN) suivant la revendication 1, **caractérisée en ce qu'**elle est bicaténaire.

3. Molécule d'ARN (siARN) suivant les revendications 1 et 2, **caractérisée en ce qu'**elle a une longueur de 21 à 25 nucléotides, de préférence une longueur de 21 nucléotides.

4. Molécule d'ARN (siARN) suivant l'une quelconque des revendications 1 à 3, **caractérisée en ce qu'**elle consiste en une des séquences choisies dans le groupe consistant en
**hTF167** 
5' g-c-g-c-u-u-c-a-g-g-c-a-c-u-a-c-a-a-a-t-t
t-t-c-g-c-g-a-a-g-u-c-c-g-u-g-a-u-g-u-u-u 5' ;
 **hTF372** 
5' g-a-a-g-c-a-g-a-c-g-u-a-c-u-u-g-g-c-a-t-t
t-t-c-u-u-c-g-u-c-u-g-c-a-u-g-a-a-c-c-g-u 5' ;
 **htf562** 
5' c-g-g-a-c-u-u-u-a-g-u-c-a-g-a-a-g-g-a-t-t
t-t-g-c-c-u-g-a-a-a-u-c-a-g-u-c-u-u-c-c-u 5' ;
 **hTF256** 
5' c-c-c-g-u-c-a-a-u-c-a-a-g-u-c-u-a-c-a-t-t
t-t-g-g-g-c-a-g-u-u-a-g-u-u-c-a-g-a-u-g-u 5' ;
 **hTF164** 
5' c-c-g-g-c-g-c-u-u-c-a-g-g-c-a-c-u-a-c-t-t
t-t-g-g-c-c-g-c-g-a-a-g-u-c-c-g-u-g-a-u-g 5' ;
 **hTF170** 
5' c-u-u-c-a-g-g-c-a-c-u-a-c-a-a-a-u-a-c-t-t
t-t-g-a-a-g-u-c-c-g-u-g-a-u-g-u-u-u-a-u-g 5' ;
 **hTF173**

5' c-a-g-g-c-a-c-u-a-c-a-a-a-u-a-c-u-g-u-t-t
t-t-g-u-c-c-g-u-g-a-u-g-u-u-u-a-u-g-a-c-a 5'.

5. Molécule d'ARN (siARN) suivant l'une quelconque des revendications 1 à 3, **caractérisée en ce qu'**une ou plusieurs mutations sont introduites par rapport à la séquence de type sauvage, ladite molécule consistant en la séquence
**hTF167-s2** 
5' g-g-g-c-u-u-c-a-g-g-c-a-c-u-a-c-a-a-a-u-a
g-c-c-c-g-a-a-g-u-c-c-g-u-g-a-u-g-u-u-u 5' ;
 ou
**htf167-s3**

5' g-c-c-c-u-u-c-a-g-g-c-a-c-u-a-c-a-a-a-u-a
g-c-c-g-g-g-a-a-g-u-c-c-g-u-g-a-u-g-u-u-u 5'.

6. Molécule d'ARN (siARN) suivant la revendication 1, **caractérisée en ce qu'**elle est modifiée en remplaçant la liaison phosphodiester par une liaison thiophosphodiester.

7. Molécule d'ARN (siARN) suivant la revendication 6, **caractérisée en ce qu'**elle est choisie dans le groupe consistant en
**hTF167-P1+1** 
5' g*c-g-c-u-u-c-a-g-g-c-a-c-u-a-c-a-a-a-u*a
g*c-c-g-c-g-a-a-g-u-c-c-g-u-g-a-u-g-u-u*u 5'
 **hTF167-P0+2** 
5' g-c-g-c-u-u-c-a-g-g-c-a-c-u-a-c-a-a-a*u*a
g*c*c-g-c-g-a-a-g-u-c-c-g-u-g-a-a-u-g-u-u-u- 5'
 **hTF167-P2+2** 
5' g*c*g-c-u-u-c-a-g-g-c-a-c-u-a-c-a-a-a*u*a
g*c*c-g-c-g-a-a-g-u-c-c-g-u-g-a-u-g-u*u*u 5'
 **hTF167-P2+4** 
5' g*c*g-c-u-u-c-a-g-g-c-a-c-u-a-c-a*a*a*u*a
g*c*c*g*c-g-a-a-g-u-c-c-g-u-g-a-u-g-u*u*u 5'
 où l'astérisque indique les nucléotides joints par des liaisons phosphorothioate.

8. Molécule d'ARN (siARN) suivant la revendication 7, **caractérisée en ce qu'**elle consiste en
**hTF167-P2+2** 
5' g*c*g-c-u-u-c-a-g-g-c-a-c-u-a-c-a-a-a*u*a
g*c*c-g-c-g-a-a-g-u-c-c-g-u-g-a-u-g-u*u*u 5',
 où l'astérisque indique les nucléotides joints par des liaisons phosphorothioate.

9. Molécule d'ARN (siARN) suivant l'une quelconque des revendications 1 à 3, **caractérisée en ce qu'**elle est modifiée par introduction d'un groupe alkyle en C₁ à C₃, alcényle en C₁ à C₃ ou alcynyle en C₁ à C₃ dans un ou plusieurs des groupes hydroxyle OH 2' dans la séquence.

10. Molécule d'ARN (siARN) suivant la revendication 9, **caractérisée en ce qu'**elle choisie dans un groupe consistant en
**hTF167-M+1** 
5' G-c-g-c-u-u-c-a-g-g-c-a-c-u-a-c-a-a-a-u-A
G-c-c-g-c-g-a-a-g-u-c-c-g-u-g-a-u-g-u-u-U 5'
 **hTF167M0+2** 
5' g-c-g-c-u-u-c-a-g-g-c-a-c-u-a-c-a-a-a-U-A
G-C-c-g-c-g-a-a-g-u-c-c-g-u-g-a-u-g-u-u-u 5'
 **hTF167-M2+2** 
5'G-C-g-c-u-u-c-a-g-g-c-a-c-u-a-c-a-a-a-U-A
G-C-c-g-c-g-a-a-g-u-c-c-g-u-g-a-u-g-u-U-U 5'
 **hTF167-M2+4** 
5' G-C-g-c-u-u-c-a-g-g-c-a-c-u-a-c-a-A-A-U-A
G-C-C-G-c-g-a-a-g-u-c-c-g-u-g-a-u-g-u-U-U 5'
 **hTF167-A1+1** 
5' G-c-g-c-u-u-c-a-g-g-c-a-c-u-a-c-a-a-a-u-A
G-c-c-g-c-g-a-a-g-u-c-c-g-u-g-a-u-g-u-u-U 5'
 **hTF167-A0+2** 
5' g-c-g-c-u-u-c-a-g-g-c-a-c-u-a-c-a-a-a-U-A
G-C-c-g-c-g-a-a-g-u-c-c-g-u-g-a-u-g-u-u-u 5',
 où une lettre en capitale en gras indique que le nucléotide est 2-O-méthylé ;
et où une lettre en capitale en gras soulignée indique que le nucléotide est 2-O-allylé.

11. Molécule d'ARN (siARN) suivant la revendication 10, **caractérisée en ce qu'**elle consiste en la séquence
**hTF167-M2+2** 
5'G-C-g-c-u-u-c-a-g-g-c-a-c-u-a-c-a-a-a-U-A
G-C-c-g-c-g-a-a-g-u-c-c-g-u-g-a-u-g-u-U-U 5'
 ou
**hTF167-M2+4** 
5' G-C-g-c-u-u-c-a-g-g-c-a-c-u-a-c-a-A-A-U-A
G-C-C-G-c-g-a-a-g-u-c-c-g-u-g-a-u-g-u-U-U 5'
 où une lettre en capitale en gras indique que le nucléotide est 2-O-méthylé.

12. Molécule d'ARN (siARN) suivant la revendication 10, **caractérisée en ce qu'**elle consiste en la séquence
**hTF167-M2+4** 
5' G-C-g-c-u-u-c-a-g-g-c-a-c-u-a-c-a-A-A-U-A
G-C-C-G-c-g-a-a-g-u-c-c-g-u-g-a-u-g-u-U-U 5',
 où une lettre en capitale en gras indique que le nucléotide est 2-O-méthylé.

13. Molécule d'ARN (siARN) suivant l'une quelconque des revendications 1 à 11, **caractérisée en ce que** le TF ou son fragment est issu d'un vertébré, avantageusement issu d'un mammifère, plus avantageusement d'origine humaine.

14. Préparation pharmaceutique, **caractérisée en ce qu'**elle comprend une ou plusieurs des molécules d'ARN interférent court (siARN) suivant l'une quelconque des revendications 1 à 13.

15. Préparation pharmaceutique suivant la revendication 14, **caractérisée en ce qu'**elle comprend la séquence
**hTF167** 
5' g-c-g-c-u-u-c-a-g-g-c-a-c-u-a-c-a-a-a-t-t
t-t-c-g-c-g-a-a-g-u-c-c-g-u-g-a-u-g-u-u-u 5' ;
 ou
**hTF372**

5' g-a-a-g-c-a-g-a-c-g-u-a-c-u-u-g-g-c-a-t-t
t-t-c-u-u-c-g-u-c-u-g-c-a-u-g-a-a-c-c-g-u 5'.

16. Préparation pharmaceutique suivant la revendication 14, **caractérisée en ce qu'**elle comprend la séquence
**hTF167-s2** 
5' g-g-g-c-u-u-c-a-g-g-c-a-c-u-a-c-a-a-a-u-a
g-c-c-c-g-a-a-g-u-c-c-g-u-g-a-u-g-u-u-u 5' ;
 ou
**htf167-s3**

5' g-c-c-c-u-u-c-a-g-g-c-a-c-u-a-c-a-a-a-u-a
g-c-c-g-g-g-a-a-g-u-c-c-g-u-g-a-u-g-u-u-u 5'.

17. Préparation pharmaceutique suivant la revendication 14, **caractérisée en ce que** lesdites molécules sont choisies dans le groupe consistant en
**hTF167-P2+2** 
5' g*c*g-c-u-u-c-a-g-g-c-a-c-u-a-c-a-a-a*u*a
g*c*c-g-c-g-a-a-g-u-c-c-g-u-g-a-u-g-u*u*u 5' ;
 **hTF167-M2+2** 
5'G-C-g-c-u-u-c-a-g-g-c-a-c-u-a-c-a-a-a-U-A
G-C-c-g-c-g-a-a-g-u-c-c-g-u-g-a-u-g-u-U-U 5' ;
 ou
**hTF167-M2+4** 
5' G-C-g-c-u-u-c-a-g-g-c-a-c-u-a-c-a-A-A-U-A
G-C-C-G-c-g-a-a-g-u-c-c-g-u-g-a-u-g-u-U-U 5',
 où l'astérisque indique les nucléotides joints par des liaisons phosphorothioate
et où une lettre en capitale en gras indique que le nucléotide est 2-O-méthylé.

18. Préparation pharmaceutique suivant la revendication 17, dans laquelle la molécule d'ARN est une molécule d'ARN isolée comprenant la séquence
**hTF167-M2+4** 
5' G-C-g-c-u-u-c-a-g-g-c-a-c-u-a-c-a-A-A-U-A
G-C-C-G-c-g-a-a-g-u-c-c-g-u-g-a-u-g-u-U-U 5',
 où une lettre en capitale en gras indique que le nucléotide est 2-O-méthylé.

19. Composition pharmaceutique suivant l'une quelconque des revendications 14 à 18, **caractérisée en ce qu'**elle comprend en outre des diluants, des lubrifiants, des liants, des supports, des moyens de délitement, des moyens d'absorption, des colorants, des agents édulcorants et/ou des agents aromatisants.

20. Composition pharmaceutique suivant l'une quelconque des revendications 14 à 19, **caractérisée en ce qu'**elle comprend des adjuvants.

21. Composition pharmaceutique suivant l'une quelconque des revendications 14 à 20, apte à l'administration par voie parentérale, de préférence par injection sous-cutanée, intraveineuse, intramusculaire ou intrapéritonéale ou par perfusion, par voie orale, nasale, buccale, rectale, ou vaginale et/ou par inhalation ou insufflation.

22. Composition pharmaceutique suivant l'une quelconque des revendications 14 à 21, **caractérisée en ce qu'**elle est formulée à l'état de solutions ou suspensions pour perfusion, d'aérosol, de capsules, de comprimés, de pilules, d'un liquide d'atomisation, de suppositoires, dans des formulations posologiques contenant des supports, adjuvants et/ou véhicules non toxiques pharmaceutiquement acceptables.

23. Utilisation d'une ou plusieurs des molécules d'ARN (siARN) suivant l'une quelconque des revendications 1 à 13 pour préparer une préparation pharmaceutique apte au traitement, à la prévention et/ou à l'inhibition d'une coagulation sanguine indésirable.

24. Utilisation suivant la revendication 23, dans laquelle ladite préparation est formulée pour l'administration parentérale, de préférence pour l'injection sous-cutanée, intraveineuse, intramusculaire ou intrapéritonéale ou la perfusion, l'administration orale, nasale, buccale, rectale, ou vaginale et/ou l'inhalation ou l'insufflation.


### Sequence listing

<110> Hans Prydz
   <120> Post transcriptional silencing by short interfering RNAs
<130> 114577/KHS
<150> NO 2002 0612 <151> 2002-02-07
<160> 31
<210>1
   <211> 21
   <212> RNA
   <213> Human Tissue-Factor DNA
   <223> si RNA
<400> 1
   gc gcu uca ggc acu aca aa tt
<210>2
   <211> 21
   <212> RNA
   <213> Human Tissue-Factor DNA
   <223> si RNA
<400> 2
   g aag cag acg uac uug gca tt
<210>3
   <211> 21
   <212> DNA
   <213> Human Tissue-Factor DNA
   <223> si RNA
<400> 3
   cgg acu uua guc aga agg a tt
<210>4
   <211> 21
   <212> RNA
   <213> Human Tissue-Factor DNA
   <223> si RNA
<400> 4
   ccc guc aau caa guc uac a tt
<210>5
   <211> 21
   <212> RNA
   <213> Human Tissue-Factor DNA
   <223> si RNA
<400> 5
   ug gcc ggc gcu uca ggc ac tt
<210>6
   <211> 21
   <212> RNA
   <213> Human Tissue-Factor DNA
   <223> si RNA
<400> 6
   cc ggc gcu uca ggc acu ac tt
<210>7
   <211> 21
   <212> RNA
   <213> Human Tissue-Factor DNA
   <223> si RNA
<400> 7
   cu uca ggc acu aca aau ac tt
<210>8
   <211> 21
   <212> RNA
   <213> Human Tissue-Factor DNA
   <223> si RNA
<400> 8
   ca ggc acu aca aau acu gu tt
<210>9
   <211> 21
   <212> RNA
   <213> syntetic
<220>
   <221> misc-difference
   <223> si RNA, comprises one base-pairing mutation compared to the wild type siRNA sequence
<400> 9
   cc gcu uca ggc acu aca aau a
<210>10
   <211> 21
   <212> RNA
   <213> Human Tissue-Factor DNA
   <223> si RNA
<210>10
   <211> 21
   <212> RNA
   <213> syntetic
<220>
   <221> misc-difference
   <223> si RNA, comprises one base-pairing mutation compared to the wild type siRNA sequence
<400> 10
   gg gcu uca ggc acu aca aau a
<210>11
   <211> 21
   <212> DNA
   <213> syntetic
<220>
   <221> misc-difference
   <223> si RNA, comprises one base-pairing mutation compared to the wild type siRNA sequence
<400> 11
   gc ccu uca ggc acu aca aau a
<210>12
   <211> 21
   <212> RNA
   <213> syntetic
<220>
   <221> misc-difference
   <223> si RNA, comprises one base-pairing mutation compared to the wild type siRNA sequence
<400> 12
   gc ggu uca ggc acu aca aau a
<210>13
   <211> 21
   <212> RNA
   <213> syntetic
<220> misc-difference
   <223> si RNA, comprises one base-pairing mutation compared to the wild type siRNA sequence
<400> 13
   gc gcu uga ggc acu aca aau a
<210>14
   <211> 21
   <212> RNA
   <213> syntetic
<220>
   <221> misc-difference
   <223> si RNA, comprises one base-pairing mutation compared to the wild type siRNA sequence
<400> 14
   gc gcu uca gcc acu aca aau a
<210>15
   <211> 21
   <212> RNA
   <213> syntetic
<220>
   <221> misc-difference
   <223> si RNA, comprises one base-pairing mutation compared to the wild type siRNA sequence
<400> 15
   gc gcu uca ggg acu aca aau a
<210>16
   <211> 21
   <212> RNA
   <213> syntetic
<210>
   <221> misc-difference
   <223> si RNA, comprises one base-pairing mutation compared to the wild type siRNA sequence
<400> 16
   gc gcu uca ggc agu aca aau a
<210>17
   <211> 21
   <212> RNA
   <213> syntetic
<220>
   <221> misc-difference
   <223> si RNA, comprises one base-pairing mutation compared to the wild type siRNA sequence <400> 17
   gc gcu uca ggc acu aga aau a
<210>18
   <211> 21
   <212> RNA
   <213> syntetic
<220>
   <221> mise-difference
   <223> si RNA, comprises two base-pairing mutation compared to the wild type siRNA sequence
<400> 18
   gc gcu uga gcc acu aca aau a
<210>19
   <211> 21
   <212> RNA
   <213> syntetic
<220>
   <221> misc-difference
   <223> si RNA, comprises two base-pairing mutation compared to the wild type siRNA sequence
<400> 19
   gc gcu uca gcg acu aca aau a
<210>20
   <211> 21
   <212> RNA
   <213> syntetic
<220>
   <221> misc-difference
   <223> si RNA, comprises two base-pairing mutation compared to the wild type siRNA sequence
<400> 20
   gc gcu uca gcc agu aca aau a
<210>21
   <211> 21
   <212> RNA
   <213> syntetic
<220>
   <221> misc-difference
   <223> si RNA, comprises two base-pairing mutation compared to the wild type siRNA sequence
<400> 21
   gc gcu uca gcc acu aga aau a
<210>22
   <211> 21
   <212> RNA
   <213> syntetic
<200>
   <221> modified-base
   <223> si RNA, the first and the last but one and last nucleotide in the 5'-3' and 3'-5'-direction are linked by phosphorothioate bonds.
<400> 22 
5'-g*c-gcuucaggcacuaca-a-a-u*a-3'
3'-g*c-c-g-cgaaguccgugaugu-u*u-5'
<210>23
   <211> 21
   <212> RNA
   <213> syntetic
   <200>
   <221> modified-base
   <223> si RNA, the last three nucleotides in the 5'-3' and the first three nucleotides in the 3'-5'-direction are linked by phosphorothioate bonds.
<400> 23 
5'-g-c-gcuucaggcacuaca-a-a*u*a-3'
3'-g*c*c-g-cgaaguccgugaugu-u-u-5'
<210>24
   <211> 21
   <212> RNA
   <213> syntetic
   <200>
   <221> modified-base
   <223> si RNA, the first and second nucleotide and the last three nucleotide in the 5'-3'-strand and the 3'-5'-strand are linked by phosphorothioate bonds.
<400> 24 
5'-g*c*gcuucaggcacuaca-a-a*u*a-3'
3'-g*c*c-g-cgaaguccgugaugu*u*u-5'
<210>25
   <211> 21
   <212> RNA
   <213> syntetic
   <200>
   <221> modified-base
   <223> si RNA, the first and second nucleotide and the last five nucleotides in the 5'-3'- and the 3'-5'-strand are linked by phosphorothioate bonds.
<400> 25 
5'-g*c*gcuucaggcacuaca*a*a*u*a-3'
3'-g*c*c*g*cgaaguccgugaugu*u*u-5'
<210>26
   <211> 21
   <212> RNA
   <213> syntetic
   <200>
   <221> modified-base, gm, um
   <223> si RNA, the first and last nucleotides in the 5'-3' direction are 2-*O*-methylated.
<400> 26 
5'-GcgcuucaggcacuacaaauA-3'
3'-GccgcgaaguccgugauguuU-5'
<210>27
   <211> 21
   <212> RNA
   <213> syntetic
   <200>
   <221> modified-base, gm, cm, um
   <223> si RNA, the last two nucleotides in both the 5'-3' and the 3'-5'-direction are 2-*O*-methylated.
<400> 27 
5’-gcgcuucaggcacuacaaaUA-3’
3’-GCcgcgaaguccgugauguuu-5’
<210>28
   <211> 21
   <212> RNA
   <213> syntetic
   <200>
   <221> modified-base, gm, cm, um
   <223> si RNA, the first, second and last two nucleotides in both the 5'-3' - and 3'-5'-direction are 2-*O-*methylated.
<400> 28 
5’-GCgcuucaggcacuacaaaUA-3’
3’-GCcgcgaaguccgugauguUU-5’
<210>29
   <211> 21
   <212> RNA
   <213> syntetic
   <200>
   <221> modified-base, gm, cm, um
   <223> si RNA, the first, second and last four nucleotides in both the 5'-3'- and 3'-5'-direction are 2-*O-*methylated.
<400> 29 
5'-GCgcuucaggcacuacaAAUA-3'

3'-GCCGcgaaguccgugauguUU-5'
<210>30
   <211> 21
   <212> RNA
   <213> syntetic
   <200>
   <221> modified-base
   <223> si RNA, the first and the last nucleotides in the both directions are 2-*O*-allylated.
<400> 
5'-Gcgcuucaggcacuacaaau A-3'
3'-Gccgcgaaguccgugauguu U-5'
<210>31
   <211> 21
   <212> RNA
   <213> syntetic
   <200>
   <221> modified-base
   <223> si RNA, the first and the last two nucleotides in both directions are 2-*O*-allylated.
<400> 31